# EUROPÄISCHE PATENTSCHRIFT

(11) **EP 1 879 844 B1**
(45) Veröffentlichungstag und Bekanntmachung des Hinweises auf die Patenterteilung: **06.04.2011**
(21) Anmeldenummer: 06742766.6
(22) Anmeldetag: 02.05.2006
(51) Int. Cl.: C07C 51/41, C07C 53/06, C07C 53/02

(54) **HERSTELLUNG VON NATRIUMDIFORMIAT**
PRODUCTION OF SODIUM DIFORMATE
PRODUCTION DE DIFORMATE DE SODIUM

(30) Priorität: 04.05.2005 DE 102005020890
(43) Veröffentlichungstag der Anmeldung: 23.01.2008
(73) Patentinhaber: BASF SE, 67056 Ludwigshafen (DE)
(72) Erfinder: HAUK, Alexander, 67067 Ludwigshafen (DE); LAUX, Gerhard, 67227 Frankenthal (DE); HEILEK, Jörg, 69245 Bammental (DE); RIECK, Daniela, 55232 Alzey (DE); LENZ, Robert, 67433 Neustadt Gimmeldingen (DE); GROPP, Stefan, 67063 Ludwigshafen (DE)
(86) Internationale Anmeldenummer: PCT/EP2006/004087
(87) Internationale Veröffentlichungsnummer: WO 2006/117187

(56) Entgegenhaltungen:
- WO-A-96/35657
- WO-A1-2004/020382
- WO-A1-2004/022517
- DE-C- 424 017

## Beschreibung

Die vorliegende Erfindung betrifft ein Verfahren zur Herstellung einer festen Natriumdiformiat-Zubereitung mit einem hohen Gehalt an Ameisensäure.

### Beschreibung

Ameisensaure Formiate besitzen eine antimikrobielle Wirkung und werden beispielsweise eingesetzt zur Konservierung sowie zur Ansäuerung von pflanzlichen und tierischen Stoffen, wie etwa von Gräsern, landwirtschaftlichen Produkten oder Fleisch, zur Behandlung von Bioabfällen oder als Additiv zur Tierernährung.

Im Bereich der Tierernährung werden als Natriumverbindungen in der Regel entweder Mischungen von Natriumdiformiat mit Trinatriumhydrogenformiat oder letzteres allein eingesetzt, siehe z.B. die WO 96/35337 und WO 04/57977. In der WO 96/35337 wird darüber hinaus über den Einsatz von Natriumdiformiat berichtet, wobei jedoch keine konkreten Angaben zur Herstellung dieser Verbindung gemacht werden.

Allgemein ist für die Verwendung von Hydrogenformiaten ein möglichst hoher Gehalt an Formiatanionen als einer der wirksamen Bestandteile wünschenswert. Aus ökonomischer Sicht ist es insbesondere vorteilhaft, wenn dieser erhöhte Gehalt an Formiatanionen mit einem möglichst hohen Anteil an Ameisensäure einher geht, da diese gleichzeitig die ansäuernde Wirkung bietet. Unter diesen Gesichtspunkten ist die Verwendung von ameisensaurem Natriumformiat besonders günstig, da bei diesem im Vergleich zu Trinatriumhydrogentetraformiat sowie gegenüber ameisensaurem Kaliumformiat jeweils ein höherer theoretischer Gehalt sowohl an Formiationen als auch an Ameisensäure vorliegt. Beide Werte sind beim Ammoniumdiformiat zwar noch etwas günstiger, jedoch handelt es sich hierbei um eine sehr unbeständige Verbindung.

Als solche sind ameisensaure Formiate in fester Form und ihre Herstellung seit langem bekannt, z.B. aus Gmelins Handbuch der anorganischen Chemie, 8. Auflage, Nummer 21, Seiten 816 bis 819, Verlag Chemie GmbH, Berlin 1928 sowie Nummer 22, Seiten 919 bis 921, Verlag Chemie GmbH, Berlin 1937. Hiernach sollen durch Lösen von Kaliumformiat bzw. Natriumformiat in Ameisensäure und anschließendes Abkühlen die sauren Formiate Kaliumdiformiat und Natriumdiformiat prinzipiell erhältlich sein. Neben Natriumdiformiat existiert die stabilere Kristallform Trinatriumhydrogentetraformiat. Es wird jedoch darauf verwiesen, dass speziell Natriumdiformiat in kristalliner, trockener Form nur schwer zugänglich und darüber hinaus relativ unbeständig ist. Die Angaben in Gmelins Handbuch lassen nur den Schluss zu, dass es sich bei den dort beschriebenen Produkten nicht um reines Natriumdiformiat handelte.

Die deutsche Patentschrift DE 424017 (vom 14.01.1926) lehrt die Herstellung von ameisensauren Natriumformiaten mit verschiedenem Säuregehalt durch Einbringen von Natriumformiat in wässrige Ameisensäure. Die dabei entstehenden Kristalle werden durch Abkühlung der Lösung auf Umgebungstemperatur erhalten. In Abhängigkeit vom Wassergehalt der Ameisensäure soll neben Trinatriumhydrogenformiat und Mischungen von Trinatriumhydrogenformiat mit Natriumdiformiat angeblich auch Natriumdiformiat zugänglich sein. Letzteres soll nach dem Verfahren der DE 424017 erhalten werden, wenn die eingesetzte Ameisensäure einen Gehalt von mehr als 50 % aufweist, z. B. 80 % wie in Beispiel 2. Eigene Versuche der Erfinder ergaben jedoch, dass sich unter den in der DE 424017 genannten Bedingungen kein Natriumdiformiat in reiner, kristalliner Form erhalten lässt. Vielmehr wird bei dieser Vorgehensweise ein Gemisch mit Trinatriumhydrogenformiat erhalten, dessen Gehalt an Ameisensäure deutlich unterhalb des für reines Natriumdiformiat erwarteten theoretischen Wertes von 40,36 Gew.-%, bezogen auf das Gesamttrockengewicht, liegt.

Die EP 0 824 511 B1 beschreibt ein Verfahren zur Herstellung von Produkten, welche Disalze von Ameisensäure enthalten. Hierbei werden bestimmte Alkali- bzw. Ammoniumformiate, -hydroxide, -(bi)carbonate oder Ammoniak bei 40 °C bis 100 °C mit Ameisensäure vermischt, die einen Gehalt von mindestens 50 % aufweist. Das Gemisch wird anschließend gekühlt und die Disalze werden durch Filtration gewonnen. Zwar wird die Herstellung von ameisensaurem Kaliumformiat sowie von Mischungen ameisensauren Natriumformiats mit Trinatriumhydrogentetraformiat exemplarisch dargelegt, die Herstellung von festem, reinem Natriumdiformiat wird hingegen nicht gelehrt. So erlauben nämlich die für das Verfahren angegebenen Temperaturen und Konzentrationsgrenzen für die einzusetzenden (wässrigen) Kalium- bzw. Natriumformiatlösungen nur die Herstellung von Kaliumdiformiat, da sich (wässrige) Lösungen von Natriumformiat aufgrund der gegenüber Kaliumformiat geringeren Löslichkeitsgrenze nicht in den angegebenen Konzentrationen herstellen lassen. Dementsprechend erhält man zwar Kaliumdiformiat, das Natriumdiformiat liegt jedoch ausschließlich in Mischung mit dem Trinatriumhydrogentetraformiat vor.

Ferner beschreibt die EP 0 824 511 B1 eine Verfahrensführung, bei der die nach der Kristallisation erhaltene Mutterlauge vollständig neutralisiert (pH = 9 bis 10) und auf einen Formiatgehalt von 70 bis 80 % eingedampft wird und bei der die so erhaltene Formiatlösung in die zur Kristallisation eingesetzte Ausgangslösung zurückgeführt wird. Um dieses in der EP 0 824 511 B1 beispielhaft anhand der Herstellung von Kaliumdiformiat dargestellte Verfahren zur Herstellung von Natriumdiformiat anwenden zu können, müsste die einzudampfende Natriumformiat-Lösung bei vergleichsweise hohen Temperaturen gehandhabt werden. So ist eine 70 gew.-%ige Natriumformiat-Lösung erst bei einer Temperatur von etwa 135 °C und eine 80 gew.-%ige Natriumformiat- Lösung erst bei einer Temperatur von 180 °C erhältlich. Derartige Temperaturen erfordern einen hohen Aufwand bei der Temperierung der eingesetzten Apparaturen, z. B. von Rohrleitungen und Armaturen. Wird nach dem Eindampfen eine 80 gew.-%ige Natriumformiat-Lösung zurückgeführt und z. B. mit 85 gew.-%iger Ameisensäurelösung abgemischt, so ist die resultierende Lösung aufgrund ihrer hohen Wasserkonzentration technisch nur mit hohem Aufwand zu kristallisieren. Die Kristallisationstemperatur einer solchen Lösung liegt unter 20 °C, so dass in der Regel ein Energiekosten- und Investitionsaufwand erforderndes Kälteaggregat notwendig ist. Darüber hinaus fällt bei der Neutralisation der gesamten Mutterlauge gemäß dem in der EP 0 824 511 B1 beschriebenen Verfahren zu viel Natriumformiat an, so dass bei Betrachtung der Gesamtbilanz ein überschüssiger Anteil ausgeschleust werden muss. Dies lässt sich auch durch den Einsatz einer höher konzentrierten Ameisensäurelösung nicht vermeiden.

Die frühere deutsche Anmeldung DE 102005017089.7 beschreibt erstmals ein Verfahren zur Herstellung von festem Natriumdiformiat mit einem Gehalt an Ameisensäure von wenigstens 35 Gew.-% in reiner, stabiler und trockener Form.

Eine ausreichende Stabilität von ameisensaurem Natriumformiat in fester Form ist sowohl hinsichtlich der Handhabung und Lagerfähigkeit als auch hinsichtlich der Herstellung von besonderer Bedeutung. Insbesondere ist eine in größerem Maße auftretende Freisetzung der in dem ameisensauren Natriumformiat enthaltenen Ameisensäure aufgrund deren korrosiver Wirkung unerwünscht.

Im Bereich der Tieremährung bietet Natriumdiformiat den Vorteil, dass das Spurenelement Natrium nicht wie sonst üblich in Form von NaCl gesondert zugesetzt werden muss, sondern bereits als solches eine Natriumquelle darstellt. Durch den hohen Gehalt an Ameisensäure im Natriumdiformiat, z. B. gegenüber Trinatriumhydrogentetraformiat, ist der Gehalt an Natriumionen begrenzt. Ein geringer bzw. begrenzter Gehalt an Kationen, z. B. auch Kaliumionen, ist insofern wünschenswert, als da letztere insbesondere bei Monogastriern und speziell bei Geflügel zu einer erhöhten Flüssigkeitsaufnahme (vermehrtem Trinken) und damit zu einer Verdünnung des Kots der Tiere führen, also eine diurethische Wirkung entfalten können.

Der vorliegenden Erfindung lag die Aufgabe zugrunde, ein Verfahren zur Herstellung einer festen, im Wesentlichen aus Natriumdiformiat bestehenden Natriumdiformiat-Zubereitung bereitzustellen, das die oben geschilderten Probleme aus dem Stand der Technik vermeidet. Insbesondere sollte eine Rückführung der Mutterlauge in das Herstellungsverfahren ermöglicht werden, ohne dass ein signifikanter Anteil an Natriumformiat ausgeschleust werden muss. Das erfindungsgemäße Verfahren sollte außerdem die Herstellung einer solchen Zubereitung ermöglichen, die einen hohen Gehalt an Ameisensäure aufweist und in der das Natriumdiformiat in hoher Reinheit sowie vergleichsweise stabiler und trockener Form vorliegt, so dass das Verfahren im Rahmen einer großtechnischen Produktion, insbesondere bei vergleichsweise geringen Temperaturen, anwendbar ist.

Diese Aufgabe wurde überraschend gelöst, indem man die Zielverbindung aus einer Mischung von Natriumformiat mit einem mehr als anderthalbfachen molaren Überschuss an Ameisensäure unter Einhaltung eines molaren Verhältnisses von Ameisensäure zu Wasser von mindestens 1,1:1 1 auskristallisiert, einen Teil der Mutterlauge direkt in die zu kristallisierende Lösung zurückführt und den anderen Teil vor der Rückführung neutralisiert.

Ein erster Gegenstand der vorliegenden Erfindung ist daher ein Verfahren zur Herstellung einer festen Natriumdiformiat-Zubereitung mit einem Gehalt an Ameisensäure von mindestens 35 Gew.-%, bezogen auf das Gesamtgewicht der Natriumdiformiat-Zubereitung, bei dem man bei erhöhter Temperatur aus Natriumformiat und wenigstens 74 gew.-%iger Ameisensäure eine wässrige Lösung herstellt, die ein molares Verhältnis von HCOOH : HCOONa von mehr als 1,5 : 1 und ein molares Verhältnis von HCOOH : H₂O von mindestens 1,1 : 1 aufweist, die wässrige Lösung zur Kristallisation bringt und die feste Phase von der Mutterlauge abtrennt, wobei man
(i) eine Teilmenge (A) der Mutterlauge bei der Herstellung der wässrigen Lösung einsetzt und
(ii) eine Teilmenge (B) der Mutterlauge mit einer Natrium-haltigen Base versetzt und das hierbei resultierende Natriumformiat enthaltende Gemisch, gegebenenfalls nach Ausschleusung eines Teils desselben und gegebenenfalls nach Konzentrierung desselben, ebenfalls bei der Herstellung der wässrigen Lösung einsetzt;
und wobei sich die Teilmengen (A) und (B) der Mutterlauge zu 100 Gew.-% ergänzen.

Die in der vorliegenden Erfindung zum Einsatz kommenden Ausgangsstoffe Natriumformiat und Ameisensäure sind kommerziell erhältlich und können als solche ohne Vorbehandlung verwendet werden.

Erfindungsgemäß wird die Mutterlauge aus der Kristallisation bei der Herstellung der wässrigen Lösung eingesetzt. Hierbei können die Teilmengen (A) und (B) der Mutterlauge die einzige im erfindungsgemäßen Verfahren eingesetzte Natriumformiatquelle darstellen. Steht jedoch (noch) keine Mutterlauge zur Verfügung, wie es z. B. vor der ersten Durchführung des Verfahrens der Fall ist, so kann, beispielsweise zu Beginn einer kontinuierlichen Verfahrensführung, z. B. technisches Natriumformiat eingesetzt werden. Auch bei der Herstellung von Polyolen als Abfallprodukt anfallendes Natriumformiat ist in diesem Fall zum Einsatz in der vorliegenden Erfindung geeignet. Ebenfalls möglich ist die Herstellung des einzusetzenden Natriumformiats z. B. durch Umsetzung von Natriumhydroxid, -carbonat oder -hydrogencarbonat mit Ameisensäure durch Umsetzung von Kohlenmonoxid mit flüssigem Natriumhydroxid oder durch Umsetzung von Methylformiat mit Natriumhydroxid. Bei dieser Variante kann man z. B. so vorgehen, dass man festes NaOH oder eine konzentrierte wässrige Lösung davon, gegebenenfalls unter Kühlung und/oder Rühren, in vorzugsweise konzentrierter Ameisensäure löst. Dabei können die Verhältnisse der Ausgangsstoffe vorteilhafterweise direkt so gewählt werden, dass die Komponenten Ameisensäure, Natriumformiat und Wasser in der resultierenden Mischung bereits in den oben genannten erforderlichen molaren Verhältnissen vorliegen. Andernfalls ist in der Regel eine Neutralisation überschüssiger Ameisensäure und/oder eine Verringerung des Wassergehalts der Mischung durch übliche, dem Fachmann bekannte Verfahren, z. B. Verdampfen, Extraktion, Destillation und dergleichen, erforderlich. In der Regel setzt man ein Natriumformiat ein, dessen Gehalt an HCOONa wenigstens 97 Gew.-%, bezogen auf das Gesamtgewicht der eingesetzten Natriumformiatquelle, beträgt. Vorzugsweise setzt man ein Natriumformiat ein, das weniger als 0,1 Gew.-% und insbesondere weniger als 0,05 Gew.-%, jeweils bezogen auf das Gesamtgewicht der eingesetzten Natriumformiatquelle, an Kaliumionen enthält. Sobald nach einem ersten Kristallisationsschritt (nachfolgend auch als Kristallisationsstufe bezeichnet) Mutterlauge zum Einsatz bei der Herstellung der wässrigen Lösung zur Verfügung steht, dienen die Teilmenge (A) und die neutralisierte Teilmenge (B) der Mutterlauge bevorzugt als alleinige Natriumformiatquelle.

Erfindungsgemäß wird eine wässrige Ameisensäurelösung mit einem Ameisensäuregehalt von wenigstens 74 Gew.-% oder eine konzentrierte Ameisensäure eingesetzt. Unter einer konzentrierten Ameisensäure versteht der Fachmann eine Ameisensäurelösung mit einem Gehalt an Ameisensäure von 94 Gew.-% oder mehr, d.h. mit einem Restwassergehalt von weniger als 6 Gew.-%, jeweils bezogen auf das Gesamtgewicht der Ameisensäurelösung. Als wässrige Ameisensäure wird eine Lösung von Ameisensäure in Wasser mit einem Ameisensäuregehalt von weniger als 94 Gew.-%, bezogen auf das Gesamtgewicht der wässrigen Ameisensäurelösung, bezeichnet. Die eingesetzte wässrige Ameisensäurelösung weist vorzugsweise eine Konzentration von wenigstens 75 Gew.-%, bevorzugt wenigstens 80 Gew.-% und besonders bevorzugt wenigstens 90 Gew.-% auf. Ganz besonders bevorzugt setzt man konzentrierte Ameisensäure mit einem Ameisensäuregehalt von wenigstens 94 Gew.-% ein. Die Konzentration der Ameisensäure bzw. Ameisensäurelösung wird vorzugsweise 99 Gew.-% nicht überschreiten und liegt besonders bevorzugt im Bereich von 80 bis 99 Gew.-% und speziell im Bereich von 94 bis 98 Gew.-%.

Vorzugsweise wird man konzentrierte oder wässrige Ameisensäure in einer Menge von mindestens 1,6 Mol, insbesondere mindestens 1,8 Mol und speziell mindestens 2,0 Mol HCOOH pro Mol HCOONa einsetzen. Vorzugsweise wird das zur Herstellung der wässrigen Lösung eingesetzte molare Verhältnis von HCOOH : HCOONa im Bereich von 1,6 : 1 bis 3 : 1 und insbesondere im Bereich von 1,8 : 1 bis 2,5 : 1 liegen.

Vorzugsweise wird das zur Herstellung der wässrigen Lösung eingesetzte molare Verhältnis von HCOOH : H₂O mindestens 1,5 : 1 und besonders bevorzugt mindestens 1,8 : 1 betragen, ganz besonders bevorzugt liegt es im Bereich von 1,5 : 1 bis 10 : 1 und insbesondere im Bereich von 1,8 : 1 bis 6,1 : 1.

Erfindungsgemäß wird die wässrige Lösung bei erhöhter Temperatur hergestellt. Hierunter versteht man in der Regel Temperaturen von wenigstens 30 °C, insbesondere wenigstens 40 °C und speziell wenigstens 50 °C, wobei in der Regel 100 °C, insbesondere 80 °C und speziell 70 °C nicht überschritten werden. Die Herstellung einer solchen wässrigen Lösung kann mit üblichen, dem Fachmann bekannten Vorgehens-weisen erfolgen, z.B. durch Vermischen, Verrühren oder Lösen unter Anwendung erhöhter Temperatur oder durch eine kombinierte Anwendung dieser Methoden. Die Reihenfolge des Einsatzes der Ausgangsstoffe ist von untergeordneter Bedeutung. Dies gilt sowohl für eine erste Durchführung des Verfahrens, bei der noch keine Mutterlauge zur Rückführung zur Verfügung steht, als auch bei Rückführung der Teilströme (A) und (B) der Mutterlauge. Vorteilhafterweise erfolgt die Vermischung derart, dass eine homogene flüssige Mischung der Ausgangsstoffe in dem einzuhaltenden molaren Verhältnis erhalten wird. Sofern diese homogene flüssige Mischung nicht bereits die wässrige Lösung darstellt, etwa weil nicht alle Komponenten vollständig gelöst vorliegen, so nimmt man die Überführung der homogenen flüssigen Mischung in die wässrige Lösung durch Erhöhung der Temperatur, vorzugsweise unter Rühren, vor.

Zur Durchführung des erfindungsgemäßen Verfahrens geht man in der Regel so vor, dass man eine wässrige oder konzentrierte, bevorzugt konzentrierte Lösung der Ameisensäure vorlegt. Zu dieser Ameisensäurelösung wird das Natriumformiat in fester Form oder in Form einer wässrigen Lösung oder Suspension zugegeben, gegebenenfalls zusammen mit weiterer Ameisensäure. Alternativ ist es auch möglich, die Einsatzstoffe in umgekehrter Reihenfolge zusammen zu geben. Wenn man im letzteren Fall festes Natriumformiat einsetzt und vorlegt, so wird man vorteilhafterweise zunächst durch Zugabe eines Teils der einzusetzenden Ameisensäure oder eines Teils der Teilmenge (A) der Mutterlauge eine rühr- bzw. pumpfähige Mischung erzeugen, zu welcher man die restliche Teilmenge der Ameisensäure gibt.

Die Teilmenge (A) der Mutterlauge aus Schritt (i) wird, vorzugsweise als Lösung in nicht aufbereiteter Form, zur Herstellung der wässrigen Lösung eingesetzt. Natürlich kann man sie auch zwischenzeitlich speichern und bedarfsgerecht zu einem späteren Zeitpunkt zur Herstellung der wässrigen Lösung einsetzen. In diesem Fall setzt man die Teilmenge (A) z.B. als Lösung oder Suspension, bevorzugt als Lösung, ein.

Das aus Schritt (ii) nach Neutralisation der Teilmenge (B) der Mutterlauge resultierende Gemisch wird in der Regel als wässrige Suspension oder als Feststoff bei der Herstellung der wässrigen Lösung eingesetzt. Gegebenenfalls wird man vor dem Einsatz zur Herstellung der wässrigen Lösung einen Teil des Gemischs ausschleusen. Das Gemisch wird vor dem Einsatz vorzugsweise teilweise oder vollständig eingedampft. Die Zugabe des Gemischs bei der Herstellung der wässrigen Lösung kann jeweils portionsweise, z. B. in 2, 3, 4 oder mehr einzelnen Portionen, die der Reaktionsmischung in einem vorgegebenen zeitlichen Abstand zueinander zugegeben werden, oder kontinuierlich, d. h. mit gleich bleibender, abnehmender oder zunehmender Geschwindigkeit, erfolgen. Während der Zugabe tritt in der Regel eine Temperaturerhöhung auf, so dass gegebenenfalls ein zusätzliches Erhitzen nicht erforderlich ist. Üblicherweise wird man die Temperatur der Mischung, z. B. durch Anpassung der Zugabegeschwindigkeit und/oder Kühlen bzw. Erhitzen der Mischung und/oder der zugegebenen Lösung, so einstellen, dass in der Mischung eine Temperatur im Bereich von 30 °C bis 80 °C und insbesondere von 40 °C bis 70 °C eingehalten wird. Vorzugsweise beträgt die Temperatur der Mischung nicht mehr als 65 °C. Erfindungswesentlich ist, dass die Kristallisation aus einer wässrigen Lösung erfolgt. Diese kann, wie unten noch weiter ausgeführt, bereits vor dem Beginn der Kristallisation mit Impfkristallen versetzt sein oder werden.

Während der Zugabe des Natriumformiats wird die Lösung oder Suspension vorteilhafterweise bewegt, z. B. gerührt. Das Bewegen wird nach Beendigung der Zugabe mindestens bis zum Erhalt einer wässrigen Lösung, im Allgemeinen bis zum Ende oder Abbruch der Kristallisation fortgesetzt.

Erfindungsgemäß kann die Vermischung der Ausgangsstoffe in allen zum Zweck der Erzeugung einer homogenen flüssigen Mischung üblicherweise verwendeten Apparaturen wie Reaktoren, Kesseln, Kolben, etc., insbesondere in Rührbehältern, speziell solchen mit innenliegenden Wärmetauscherflächen, durchgeführt werden. Diese sind dem Fachmann bekannt. Zur Vermeidung von Korrosionseffekten, z. B. bei Reaktoren oder Kesseln aus Stahl, ist es vorteilhaft, wenn die mit Ameisensäure in Kontakt kommenden Flächen und Wände mit einer säurebeständigen Schutzschicht, z. B. aus Teflon^{®}, beschichtet sind oder mit speziell säurebeständigen hochlegierten Stählen ausgekleidet sind.

Anschließend wird die wässrige Lösung, vorzugsweise unter fortgesetztem Rühren, zur Kristallisation gebracht. Dies kann z. B. durch teilweises Verdampfen oder durch Abkühlen, bevorzugt durch Abkühlen bewirkt werden. Wird die Kristallisation durch ein kontrolliertes Abdampfen der flüssigen Phase, vorzugsweise unter Vakuum, bewirkt oder eingeleitet bzw. beschleunigt, so ist sicherzustellen, dass die molaren Verhältnisse der Komponenten in der Lösung zu Beginn der Kristallisation innerhalb der oben spezifizierten Bereiche liegen. Wird die Kristallisation durch Abkühlen bewirkt, so erfolgt dieses vorzugsweise langsam, vorteilhafterweise über einen Zeitraum von ein bis mehreren Stunden, z. B. bis 12 h, insbesondere von 3 bis 10 h und speziell von 4 bis 8 h. Hierbei kristallisiert das Natriumdiformiat aus. Es hat sich als vorteilhaft erwiesen, wenn die Abkühlung mit einer Abkühlrate im Bereich von etwa 2 bis etwa 20 K/h, z. B. etwa 5 bis 15 K/h, erfolgt. Um eine weitgehende Kristallisation der Zielverbindung zu erzielen, ist es von Vorteil, die wässrige Lösung im genannten Zeitraum auf eine Temperatur von weniger als 20 °C, z.B. etwa 15 °C oder weniger oder 10 °C oder weniger, abzukühlen. In der Regel wird hierbei eine Temperatur von 0 °C und insbesondere von 5 °C nicht unterschritten.

Es hat sich als vorteilhaft erwiesen, nach Einsetzen der Kristallbildung die zunächst gebildeten Kristallkeime bzw. kleinen Kristalle durch Erhitzen z. B. auf eine Temperatur von maximal 65 °C, insbesondere im Bereich von 25 °C bis 50 °C, aufzulösen und den Kristallisationsvorgang anschließend durch erneutes, gegebenenfalls verlangsamtes Abkühlen wieder einsetzen zu lassen. Bei diesem erneuten Abkühlen liegt die Geschwindigkeitsrate üblicherweise im Bereich von etwa 0,5 bis etwa 20 K/h, z. B. bei etwa 1 bis 15 K/h, insbesondere bei etwa 2 bis 15 K/h, speziell bei etwa 5 bis 10 K/h und bevorzugt bei höchstens 25 K/h. Die Kristallisationstemperatur liegt in den oben genannten Bereichen.

Weiterhin kann es vorteilhaft sein, der wässrigen Lösung bereits vorhandene, z. B. durch das erfindungsgemäße Verfahren zuvor hergestellte Kristalle von Natriumdiformiat zur Förderung des Kristallisationsvorgangs, d. h. zum Zweck des so genannten "Animpfens", zuzugeben. Derartige Kristalle können in trockener oder feuchter Form, suspendiert in einer flüssigen, z. B. wässrigen oder ameisensauren, Phase oder einer Kombination dieser Formen zugegeben werden. Hierbei erfolgt die Zugabe in der Regel oberhalb einer Temperatur, die zu einer Kristallbildung führt, jedoch unterhalb einer Temperatur, bei der eine homogene Lösung vorliegt. Die Temperatur der Reaktionsmischung wird daher bei der Zugabe von Kristallen in der Regel 65 °C nicht überschreiten und vorzugsweise im Bereich von 25 bis 50 °C liegen. Der Kristallisationsvorgang kann dann, wie zuvor beschrieben, mit einer Abkühlrate im Bereich von etwa 0,5 bis etwa 20 K/h, z.B. etwa 1 bis 15 K/h, insbesondere etwa 2 bis 15 K/h und speziell etwa 5 bis 10 K/h, erfolgen. Die Kristallisationstemperatur liegt in den oben genannten Bereichen.

Im Anschluss an die Kristallisation trennt man das erhaltene feste Produkt von der Mutterlauge ab. Die Abtrennung der festen Phase von der Mutterlauge kann durch hierzu übliche, dem Fachmann bekannte Verfahren, z. B. Filtration oder Zentrifugation, vorzugsweise durch Zentrifugation, insbesondere unter Einsatz von Schub- oder Schälzentrifugen, vorgenommen werden. Die so gewonnene feuchte Natriumdiformiat-Zubereitung enthält in der Regel noch geringe Mengen an Ameisensäure, Wasser und/oder Natriumformiat. Der Gehalt an Ameisensäure in dieser noch feuchten Natriumdiformiat-Zubereitung liegt üblicherweise bei mehr als 40,3 Gew.-% und insbesondere im Bereich von 40,7 bis 42,5 Gew.-%, bezogen auf das gesamte Gewicht der feuchten Zubereitung.

Das feuchte Produkt wird anschließend durch übliche Trocknungsverfahren, z. B. unter Vakuum und/oder mäßigem Erhitzen, getrocknet. Hierfür einsetzbare Trockner und Trocknungsverfahren sind dem Fachmann bekannt und z. B. in K. Kröll, Trockner und Trocknungsverfahren, 2. Aufl., Springer Verlag, Berlin 1978 beschrieben. Insbesondere können z. B. Kontakttrockner, Wirbelschichttrockner, Sprühtrockner und Strahlungstrockner verwendet werden. Dabei ist die relativ leichte Flüchtigkeit der im Produkt enthaltenen Ameisensäure sowie die begrenzte Temperaturstabilität des Produkts zu berücksichtigen. Während der Trocknung wird man in der Regel eine Produkttemperatur von 65 °C und insbesondere 50 °C nicht überschreiten. Der nach der Trocknung im Produkt verbleibende Wassergehalt (Restwassergehalt) beträgt in der Regel nicht mehr als 0,5 Gew.-% und liegt üblicherweise im Bereich von etwa 0,5 bis 0,01 Gew.-%, bevorzugt bei höchstens 0,3 Gew.-%, besonders bevorzugt höchstens 0,2 Gew.-% und ganz besonders bevorzugt höchstens 0,1 Gew.-%, bezogen auf das Gesamtgewicht, bestimmt durch oxidimetrische Titration nach Karl Fischer (z. B. beschrieben in Wiland, Wasserbestimmung durch Karl-Fischer-Titration, Darmstadt, GIT, 1985).

Hier und im Folgenden wird der Ausdruck Gesamtgewicht der Natriumdiformiat-Zubereitung synonym mit dem Ausdruck Gesamttrockengewicht verwendet. Das Gesamttrockengewicht ist als das Gewicht der Natriumdiformiat-Zubereitung zu verstehen, das sich durch Trocknen des Produkts unterhalb seiner Zersetzungstemperatur ergibt, z. B. durch Trocknen über einen Zeitraum von 1 h bei einer Temperatur von 35 °C und einem Druck von 50 mbar.

Für die Durchführung des erfindungsgemäßen Verfahrens ist es vorteilhaft, bei der Kristallisation des Natriumdiformiats eine möglichst hohe Ausbeute zu erzielen, weil dadurch die internen Stoffströme minimiert werden können. Hierdurch kann der apparative Aufwand reduziert werden, indem z. B. die eingesetzten Apparaturen geringer dimensioniert werden können.

Die im Anschluss an die Kristallisation abgetrennte Mutterlauge wird erfindungsgemäß in zwei Teilmengen (A) und (B) aufgeteilt. Die Teilmenge (A) wird mit der Ameisensäurelösung und mit der gemäß Schritt (ii) aufgearbeiteten Teilmenge (B), gegebenenfalls nach teilweiser oder vollständiger Vermengung der genannten Stoffströme, in die Kristallisationsstufe zurückgeführt. Hierbei können die Teilmengen (A) und (B) in üblichen Behältern wie Tanks oder Kesseln zwischengelagert werden, wodurch die Dosierung bedarfsgerecht gesteuert werden kann. Das Gewichtsverhältnis von Teilmenge (A) zu Teilmenge (B) der Mutterlauge liegt bevorzugt im Bereich von 20:1 bis 1:10, noch bevorzugter im Bereich von 10:1 bis 1:5, besonders bevorzugt im Bereich von 8:1 bis 1:2 und ganz besonders bevorzugt im Bereich von 5:1 bis 1:1. Aus der entsprechenden molaren Konzentration der Ameisensäure in den Teilmengen (A) und (B) lassen sich die einzustellenden Volumenströme festlegen.

Die Teilmenge (B) wird einer Neutralisationsstufe zugeführt, in der eine teilweise oder vollständige Neutralisation erfolgt. Hierfür geeignete Natrium-haltige Basen sind Natriumhydroxid, Natriumcarbonat, Natriumhydrogencarbonat, Natrium-C₁-C₆-alkanolate, wie Natriummethanolat, -ethanolat, -propanolat, -butanolat, -pentanolat und -hexanolat, und Mischungen davon. Vorzugsweise ist die Base ausgewählt unter Natriumhydroxid, Natriumcarbonat und Mischungen davon. Die Basen können z. B. in Form einer wässrigen Lösung eingesetzt werden. Vorzugsweise versetzt man die Teilmenge (B) mit einer Natriumhydroxid- und/oder Natriumcarbonat-haltigen Lösung, z. B. einer 50 gew.-%igen Natriumhydroxidlösung, einer 20 bis 30 gew.-%igen Natriumcarbonatlösung oder einer Mischung davon. Vorzugsweise wird man die Teilmenge (B) im Wesentlichen vollständig neutralisieren. Im Wesentlichen vollständige Neutralisation bedeutet hier, dass die eingesetzte Menge der verwendeten Base mindestens der in der Teilmenge (B) vorliegenden Ameisensäure entspricht und insofern theoretisch zur vollständigen Neutralisation ausreicht.

Von dem bei der Neutralisation resultierenden Natriumformiat enthaltenden Gemisch entnimmt man gegebenenfalls einen Teil und schleust diesen aus. Dies kann erforderlich sein, um einen bei Betrachtung der Gesamtbilanz bei der Neutralisation anfallenden Überschuss an Natriumformiat zu entfernen. Für das erfindungsgemäße Verfahren ist es bevorzugt, den Anteil an auszuschleusendem Natriumformiat möglichst gering zu haften, um eine optimale Verfahrensführung und Produktausbeute zu ermöglichen. In der Regel wird die ausgeschleuste Menge höchstens 20 Gew.-%, insbesondere höchstens 10 Gew.-% und speziell höchstens 5 Gew.-% des Natriumformiat enthaltenden Gemischs, bezogen auf das Gesamtgewicht des Gemischs, betragen. Vorzugsweise schleust man nur einen solchen Teil des Gemischs aus, dass die im verbleibenden Teil des Gemischs enthaltene Menge Natriumformiat zusammen mit der in der Teilmenge (A) der Mutterlauge enthaltenen Menge Natriumformiat die zur Herstellung der wässrigen Lösung eingesetzte Gesamtmenge Natriumformiat ergibt (d.h. der Einsatz zusätzlichen Natriumformiats ist nicht erforderlich).

Den nicht entnommenen Teil des bei der Neutralisation resultierenden Natriumformiat enthaltenden Gemischs führt man einer Konzentrierungsstufe, vorzugsweise einer Eindampfungsstufe, zu. In dieser trägt man einen Teil des in dem Gemisch enthaltenen Wassers aus, vorzugsweise durch Eindampfen. Der Anteil des hierbei ausgetragenen Wassers hängt davon ab, in welcher Form das in dem Gemisch enthaltene Natriumformiat in die Kristallisationsstufe zurückgeführt werden soll. Dies kann z. B. in Form einer Lösung, Suspension oder als Feststoff erfolgen. Vorzugsweise erfolgt die Rückführung als pumpfähige Suspension oder als Feststoff, der gegebenenfalls noch Anteile von Restfeuchte aufweist. Das der Konzentrierungsstufe entnommene und rückgeführte Natriumformiat-haltige Gemisch weist in der Regel einen Gehalt an Natriumformiat von mindestens 50 Gew.-%, insbesondere mindestens 60 Gew.-%, speziell im Bereich von 50 bis 100 Gew.-% und ganz speziell im Bereich von 70 bis 90 Gew.-%, jeweils bezogen auf das Gesamtgewicht des zurückgeführten Gemischs, auf. Der Wassergehalt des Gemischs beträgt vorzugsweise höchstens 25 Gew.-% und besonders bevorzugt höchstens 15 Gew.-%, jeweils bezogen auf das Gesamtgewicht des Gemischs. Das erhaltene bzw. aufgearbeitete Natriumformiat-haltige Gemisch führt man in die Kristallisationsstufe zurück.

Die Verringerung des Wassergehalts kann auch mittels einer zweiten Kristallisationsstufe, in der man eine zweite feste Phase und eine zweite Mutterlauge erhält, und einer zweiten Konzentrierungsstufe, in der man die zweite feste Phase von der zweiten Mutterlauge abtrennt, erfolgen. Hierbei handelt es sich bei der zweiten festen Phase um Natriumformiat. Dessen Kristallisationsbedingungen sind dem Fachmann bekannt und z. B. in Zagidullin, S. K., et al., "Investigation of Supersaturations in the Sodium Formate - Water System to Optimize Crystallization", Russian Journal of Applied Chemistry, Vol. 69 (1996), 5, 669-672 beschrieben. Beispielsweise kann eine Verdampfungskristallisation oder eine Kühlungskristallisation mittels Wand- oder Siedekühlung durchgeführt werden. Zu beachten ist, dass bei tiefen Temperaturen, z. B. bei weniger als 30 °C oder weniger als 20 °C, Hydratformen des Natriumformiats, die mehr als ein als Kristallwasser gebundenes H₂O-Molekül pro Natriumformiat-Einheit aufweisen, auskristallisieren können. Dies ist in der Regel unerwünscht und daher insbesondere durch Kristallisation bei höheren Temperaturen zu vermeiden.

Die hierbei resultierende zweite feste Phase kann einen geringen Wassergehalt von weniger als 15 Gew.-%, insbesondere weniger als 10 Gew.-% und speziell weniger als 5 Gew.-% aufweisen. Ein spezieller Vorteil dieses niedrigen Wassergehalts liegt darin, dass die Kristallisation des Natriumdiformiats bei niedrigen Wassergehalten, z. B. bei weniger als 10 Gew.-%, bezogen auf die wässrige Lösung, die zur Kristallisation gebracht wird, durchgeführt werden kann. Dadurch sind höhere Kristallisationstemperaturen sowie höhere Ausbeuten bei fixer Endtemperatur realisierbar.

Das erfindungsgemäße Verfahren kann besonders vorteilhaft durchgeführt werden, wenn die in der Teilmenge (B) enthaltene molare Menge Ameisensäure in etwa der mit dem Produktstrom ausgeschleusten molaren Menge Natriumdiformiat (und gegebenenfalls Natriumformiat, das insbesondere aufgrund von Restfeuchte dem Produkt anhaften kann) entspricht oder nur unwesentlich darüber liegt, d. h. wenn ein molares Verhältnis dieser Komponenten von etwa 1:1 vorliegt. In diesem Fall ist es nämlich auf einfache Weise möglich, das gesamte einzusetzende Natriumformiat über die rückgeführten Stoffströme zu gewinnen, ohne dass eine Ausschleusung überschüssigen Natriumformiats notwendig wäre. Ein Teil des Natriumformiats wird hierbei über die Rückführung der Mutterlauge erneut in das Verfahren eingespeist. Der restliche Teil kann hierbei vollständig durch die Rückführung der neutralisierten bzw. aufkonzentrierten Teilmenge (B) der Mutterlauge bewirkt werden. Zur Durchführung dieser Verfahrensvariante wird man in der Regel das Mengenverhältnis von Teilmenge (A) zu Teilmenge (B) der Mutterlauge so einstellen, dass das molare Verhältnis von HCOOH in der Teilmenge (B) der Mutterlauge zu der Gesamtstoffmenge des in der erhaltenen festen Phase enthaltenen Natriumdiformiats und gegebenenfalls enthaltenen Natriumformiats, vor einer gegebenenfalls anschließenden Trocknung der festen Phase höchstens 1,2 : 1, bevorzugt höchstens 1,1 : 1 und besonders bevorzugt höchstens 1,05 : 1 beträgt.

In einer bevorzugten Ausführungsform geht man so vor, dass man
a) einen Strom (1) der Ameisensäure mit einem Ameisensäuregehalt von wenigstens 74 Gew.-% bereitstellt;
b) den Strom (1) aus Schritt a) mit zwei das Natriumformiat enthaltenden Strömen (5a) und (10) einer Kristallisationsstufe zuführt, wobei man, gegebenenfalls unter Temperaturerhöhung, die wässrige Lösung, die ein molares Verhältnis von HCOOH : Na[HCOO] von mehr als 1,5 : 1 und ein molares Verhältnis von HCOOH : H₂O von mindestens 1,1 : 1 aufweist, erhält;
c) in der Kristallisationsstufe die wässrige Lösung aus Schritt b) unter Erhalt eines die feste Phase und die Mutterlauge aufweisenden Stroms (3) zur Kristallisation bringt;
d) den Strom (3) aus Schritt c) einer Trennungsstufe zuführt, in der man die feste Phase von der Mutterlauge abtrennt, wobei man einen das Natriumdiformiat enthaltenden Strom (4) und einen die Mutterlauge enthaltenden Strom (5) erhält;
e) den Strom (5) aus Schritt d) in zwei Teilströme (5a) und (5b) aufteilt;
f) den Strom (5a) aus Schritt e) als Teilmenge (A) in den Schritt b) zurückführt;
g) den Strom (5b) aus Schritt e) als Teilmenge (B) mit einem die Natrium-haltige Base enthaltenden Strom (6) einer Neutralisationsstufe zuführt, wobei das Natriumformiat enthaltende Gemisch resultiert; und
h) das Natriumformiat enthaltende Gemisch aus Schritt g), gegebenenfalls nach Entnahme eines Teils desselben in Form des Stroms (7a), als Strom (7) einer Konzentrierungsstufe zuführt, in der man einen Teil des im Strom (7) enthaltenen Wassers als Strom (9) austrägt, wobei man den Natriumformiat enthaltenden Strom (10) erhält, den man in den Schritt b) zurückführt.

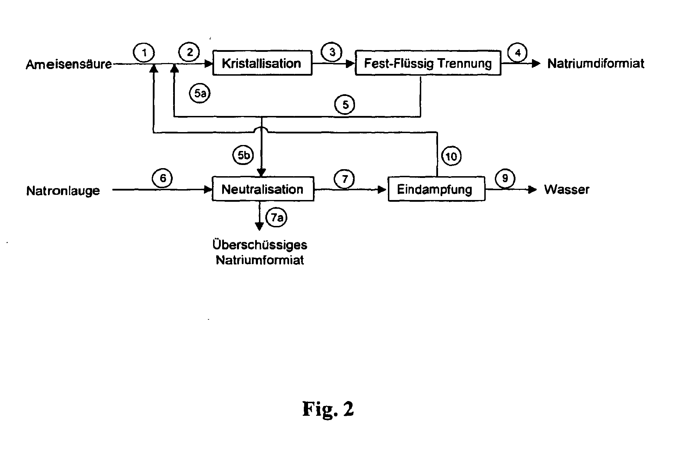

Eine dieser bevorzugten Ausführungsform entsprechende schematische Darstellung des Verfahrens ist in der beigefügten Fig. 2 wiedergegeben. Das Vermengen der Ströme (5a) und (10) mit dem Strom (1) in Schritt b) kann vor oder nach Zufuhr zu der Kristallisationsstufe erfolgen, z.B. vor der Zufuhr derart, dass dem Strom (1) zunächst der Strom (10) und anschließend der Strom (5a) zugeführt wird. Natürlich kann man die Ströme (5a) und (10) auch vor der Zusammenführung mit dem Strom (1) oder vor Zufuhr zu der Kristallisationsstufe miteinander vermengen.

Üblicherweise enthält bei dieser Ausführungsform im Schritt d) der Strom (5) im Wesentlichen Ameisensäure im Bereich von 35 bis 80 Gew.-%, besonders bevorzugt im Bereich von 40 bis 75 Gew.-%; Natriumformiat im Bereich von 20 bis 45 Gew.-%, besonders bevorzugt im Bereich von 20 bis 40 Gew.-%; und Wasser im Bereich von 0 bis 30 Gew.-%, besonders bevorzugt im Bereich von 5 bis 25 Gew.-%; jeweils bezogen auf das Gesamtgewicht des Stroms (5). Im Schritt g) setzt man als Strom (6) vorzugsweise eine wässrige Natriumhydroxid-, Natriumcarbonat- und/oder Natriumhydrogencarbonat-haltige Lösung ein. Besonders bevorzugt ist eine wässrige Natronlauge mit einem Gehalt an NaOH im Bereich von 10 bis 60 Gew.-% und bevorzugt im Bereich von 20 bis 55 Gew.-%, jeweils bezogen auf das Gesamtgewicht der wässrigen Natronlauge. Der Strom (10) aus Schritt h) enthält üblicherweise im Wesentlichen Natriumformiat im Bereich von 50 bis 100 Gew.-%, bevorzugt im Bereich von 55 bis 95 Gew.-% und besonders bevorzugt im Bereich von 70 bis 90 Gew.-%; und Wasser im Bereich von 0 bis 50 Gew.-%, bevorzugt im Bereich von 5 bis 45 Gew.-% und besonders bevorzugt im Bereich von 10 bis 30 Gew.-%, jeweils bezogen auf das Gesamtgewicht des Stroms (10).

Der Ausdruck "im Wesentlichen" bedeutet hier, dass keine signifikanten Anteile anderer Stoffe im jeweiligen Strom enthalten sind. Zum Beispiel können im Strom (5) geringe Anteile kleiner Natriumdiformiatkristalle enthalten sein, die bei der Phasentrennung in der Trennstufe nicht mit abgetrennt wurden oder die sich nach Abtrennung neu gebildet haben. In der Regel wird der Anteil weiterer Stoffe in den Strömen (5) und (10) jedoch nicht mehr als 5 Gew.-% und insbesondere nicht mehr als 3 Gew.-% betragen.

In einer weiteren bevorzugten Ausführungsform führt man den Natriumformiat enthaltenden Strom (10) aus Schritt h) vor der Rückführung in den Schritt b) einer zweiten Kristallisationsstufe und einer zweiten Trennungsstufe zu. Im einzelnen geht man so vor, dass man
k) den Natriumformiat enthaltenden Strom (10) aus Schritt h) vor der Rückführung in den Schritt b) einer zweiten Kristallisationsstufe zuführt und hierin unter Erhalt einer zweiten festen Phase und einer zweiten Mutterlauge zur Kristallisation bringt;
I) die aus Schritt k) erhaltene zweite feste Phase und zweite Mutterlauge in Form eines Stroms (12) einer Trennungsstufe zuführt, in der man die zweite feste Phase von der zweiten Mutterlauge abtrennt, wobei man einen die zweite Mutterlauge enthaltenden Strom (13) und einen Natriumformiat enthaltenden Strom (14) erhält;
m) den Natriumformiat enthaltenden Strom (14) aus Schritt I) in den Schritt b) zurückführt und hierin als Strom (10) verwendet; und
n) den die Mutterlauge enthaltenden Strom (13) aus Schritt I)
   n1) in den Schritt h) zurückführt und hierin mit dem Strom (7) der Konzentrierungsstufe des Schritts h) zuführt;
   n2) in den Schritt k) zurückführt und hierin mit dem Strom (10) der zweiten Kristallisationsstufe zuführt;
   n3) in die Teilströme (13a) und (13b) aufteilt, den Teilstrom (13a) in den Schritt h) zurückführt und hierin mit dem Strom (7) der Konzentrierungsstufe des Schritts h) zuführt und den Teilstrom (13b) in den Schritt k) zurückführt und hierin mit dem Strom (10) der zweiten Kristallisationsstufe zuführt; und/oder
   n4) teilweise entnimmt und ausschleust.

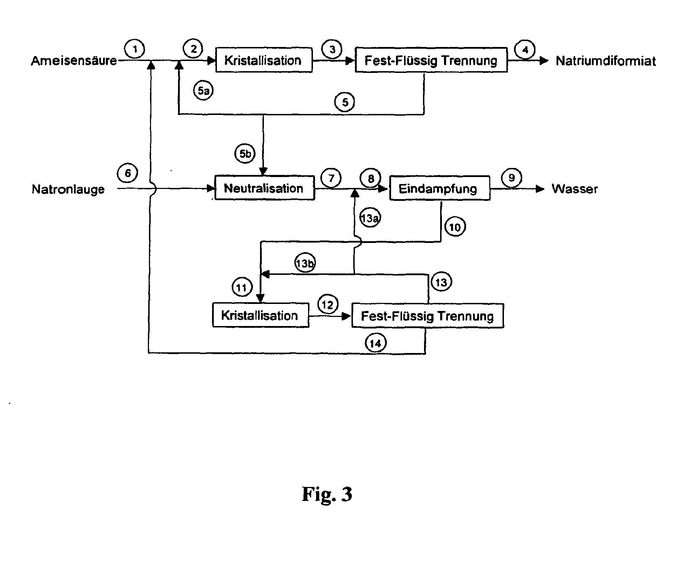

Eine dieser bevorzugten Ausführungsform entsprechende schematische Darstellung des Verfahrens ist in den beigefügten Fig. 3, 4 und 5 wiedergegeben.

Üblicherweise enthält in dieser Ausführungsform der die Mutterlauge enthaltende Strom (13) aus Schritt I) im Wesentlichen Wasser im Bereich von 20 bis 60 Gew.-%, bevorzugt im Bereich von 25 bis 55 Gew.-% und besonders bevorzugt im Bereich von 30 bis 50 Gew.-%; und Natriumformiat im Bereich von 40 bis 80 Gew.-%, bevorzugt im Bereich von 45 bis 75 Gew.-% und besonders bevorzugt im Bereich von 50 bis 70 Gew.-%, jeweils bezogen auf das Gesamtgewicht des Stroms (13). Den die Mutterlauge enthaltenden Strom (13) aus Schritt I) führt man vorzugsweise entweder gemäß Schritt n1) in den Schritt h) zurück oder gemäß Schritt n2) in den Schritt k) zurück. Im Schritt h) kann der rückgeführte Strom (13) mit dem Strom (7) unter Erhalt eines Stroms (8) zusammengeführt werden; den Strom (8) führt man dann der Konzentrierungsstufe des Schritts h) zu. Natürlich können der Strom (7) und der rückgeführte Strom (13) auch separat der Konzentrierungsstufe zugeführt werden. Im Schritt k) kann der rückgeführte Strom (13) mit dem Strom (10) unter Erhalt eines Stroms (11) zusammengeführt werden; den Strom (11) führt man dann der zweiten Kristallisationsstufe des Schritts k) zu. Natürlich können der Strom (10) und der rückgeführte Strom (13) auch separat der zweiten Kristallisationsstufe zugeführt werden. Gegebenenfalls entnimmt man den Strom (13) aus Schritt I) teilweise gemäß Schritt n4) und schleust diesen entnommenen Teilstrom aus. Hierbei wird man in der Regel höchstens 30 Gew.-%, insbesondere höchstens 20 Gew.-% und speziell höchstens 10 Gew.-% des Stroms (13), bezogen auf das Gesamtgewicht des Stroms (13), entnehmen und ausschleusen. In der Regel wird man den die Mutterlauge enthaltenden Strom (13) aus Schritt I) nur insoweit teilweise ausschleusen, wie dies z.B. zur Regulierung der Stoffbilanz, insbesondere des Wassergehalts erforderlich wird. Vorzugsweise entnimmt man im Schritt n) keinen Teil des Stroms (13) gemäß Schritt n4). Üblicherweise enthält der Natriumformiat enthaltende Strom (14) aus Schritt I) im Wesentlichen Natriumformiat im Bereich von 75 bis 100 Gew.-%, insbesondere im Bereich von 90 bis 99 Gew.-% und speziell im Bereich von 95 bis 98 Gew.-%; und Wasser im Bereich von 0 bis 25 Gew.-%, insbesondere im Bereich von 1 bis 10 Gew.-% und speziell im Bereich von 2 bis 5 Gew.-%, jeweils bezogen auf das Gesamtgewicht des Stroms (14).

Der Ausdruck "im Wesentlichen" bedeutet hier, dass keine signifikanten Anteile anderer Stoffe im jeweiligen. Strom enthalten sind. In der Regel wird der Anteil weiterer Stoffe in den Strömen (13) und (14) nicht mehr als 5 Gew.-% und insbesondere nicht mehr als 3 Gew.-% betragen.

Das erfindungsgemäße Verfahren kann kontinuierlich, halbkontinuierlich oder batchweise durchgeführt werden.

Die feste Natriumdiformiat-Zubereitung wird durch das erfindungsgemäße Verfahren in hoher Reinheit erhalten und weist daher nach Trocknung einen hohen Gehalt an Ameisensäure, in der Regel mindestens 35 Gew.-%, häufig wenigstens 36 Gew.-%, insbesondere wenigstens 37 Gew.-%, speziell wenigstens 38 Gew.-%, ganz speziell wenigstens 39 Gew.-% und noch spezieller wenigstens 40 Gew.-%, jeweils bezogen auf das Gesamtgewicht der Natriumdiformiat-Zubereitung, auf. In der Regel wird der Gehalt an Ameisensäure in der erfindungsgemäß erhaltenen Natriumdiformiat-Zubereitung nicht mehr als 41 Gew.-% und insbesondere nicht mehr als 40,5 Gew.-%, jeweils bezogen auf das Gesamtgewicht, betragen. Speziell liegt der Gehalt im Bereich von 38 bis 41 Gew.-%, ganz speziell im Bereich von 39 bis 40,5 Gew.-% und noch spezieller im Bereich von 40 bis 40,3 Gew.-%, jeweils bezogen auf das Gesamtgewicht der erhältlichen Natriumdiformiat-Zubereitung. Der Gehalt an Ameisensäure im trockenen Produkt kann in üblicher Weise, z. B. durch Titration der Ameisensäure mit einer Base, bestimmt werden. Es liegt naturgemäß ebenfalls ein hoher Gehalt an Formiatanionen im trockenen Produkt vor.

Die erfindungsgemäß erhaltene Natriumdiformiat-Zubereitung wird typischerweise in kristalliner Form erhalten. Es wird angenommen, dass die Zubereitung im Wesentlichen oder vollständig der Formel HCOONa · HCOOH (Natriumdiformiat) entspricht, was jedoch nicht als Einschränkung der Erfindung zu verstehen ist. Erfindungswesentlich ist vielmehr, dass die Zubereitung Natriumformiat und Ameisensäure in assoziierter, kristalliner Form aufweist. Die erfindungsgemäß erhaltene kristalline Modifikation des Natriumdiformiat lässt sich beispielsweise über Röntgenweitwinkelstreuung nachweisen. Unerwünschte Modifikationen, z. B. Trinatriumhydrogentetraformiat, können nach derselben Methode ebenfalls qualitativ detektiert werden. Das molare Verhältnis der Komponenten Natriumformiat und Ameisensäure in der Zubereitung liegt üblicherweise im Bereich von 0,9 :1 bis 1,1 : 1; insbesondere im Bereich von 0,95 : 1 bis 1,05 : 1 und entspricht speziell etwa 1 : 1. Der Anteil an Natriumdiformiat in der Zubereitung beträgt üblicherweise mindestens 97 Gew.-%, insbesondere mindestens 98 Gew.-% und speziell mindestens 99 Gew.-%, jeweils bezogen auf das Gesamtgewicht der Zubereitung. Als weitere Bestandteile kann die Zubereitung aufgrund von Restfeuchte oder kristallisierter Restfeuchte in der Regel bis zu 1,5 Gew.-% Ameisensäure, bis zu 1,5 Gew.-% Natriumformiat und/oder bis zu 0,5 Gew.-% Wasser enthalten, jeweils bezogen auf das Gesamtgewicht der Zubereitung. Bei etwa 65 °C ist mittels DSC (dynamische Differenzkalorimetrie) ein Phasenumwandlungspunkt zu beobachten. Die Zubereitung zeichnet sich durch eine vergleichsweise geringe Hygroskopizität aus, insbesondere im Vergleich zu Trinatriumhydrogentetraformiat. Außerdem ist die erfindungsgemäß erhaltene Natriumdiformiat-Zubereitung hinreichend stabil, um eine unproblematische Handhabung und (Weiter-)Verarbeitung zu gewährleisten. Darüber hinaus beträgt der Gehalt an Kaliumionen der erhaltenen Zubereitung in der Regel höchstens 1000 ppm und insbesondere höchstens 500 ppm, jeweils bezogen auf das Gesamtgewicht. Herstellungsbedingt liegt der Chloridgehalt in der erfindungsgemäß erhaltenen Natriumdiformiat-Zubereitung in der Regel weniger als 1500 ppm und insbesondere weniger als 1000 ppm, jeweils bezogen auf das Gesamtgewicht.

Das erfindungsgemäße Verfahren zur Herstellung einer festen, trockenen Natriumdiformiat-Zubereitung in kristalliner, stabiler Form ermöglicht eine Übertragbarkeit der Herstellungsbedingungen auf einen großtechnischen Maßstab. Es zeichnet sich insbesondere dadurch aus, dass ein effizienter Weg zur Ausschleusung von Wasser realisiert wird. Hierdurch kann speziell der Wassergehalt der zu kristallisierenden wässrigen Lösung gering gehalten werden, was die oben genannten Vorteile mit sich bringt.

Das erhaltene feste Produkt kann vor und/oder nach dem Trocknungsschritt zerkleinert werden, z. B. mittels Mörsern, Schneidgeräten, Lochpressen und Walzenstühlen, agglomeriert werden, z. B. mittels Mischern, und/oder kompaktiert werden, z. B. mittels Pressen und Kompaktoren. Die für eine derartige Zerkleinerung eingesetzten Apparaturen sind dem Fachmann bekannt.

Je nach gewünschtem Anwendungszweck kann die erfindungsgemäß hergestellte Natriumdiformiat-Zubereitung weiter verarbeitet werden, insbesondere können Pulver bestimmter Teilchengrößen erzeugt werden, die erzeugten Partikel mit Überzügen beschichtet werden und/oder Mischungen mit weiteren Zusatzstoffen hergestellt werden. Als Beispiele für Überzüge bzw. Coatingmaterialien seien Öle wie Sojaöl, Fette und Fettsäuren wie Palmitin- oder Stearinsäure oder Polymerüberzüge z. B. aus Polyalkylenen und Derivaten davon, genannt. Übliche Zusatzstoffe sind insbesondere Fließhilfsmitel wie Kieselsäure etc. Zur Beschichtung übliche Verfahren sowie die dabei in Betracht kommenden Zusatzstoffe sind dem Fachmann auf dem jeweiligen Gebiet grundsätzlich bekannt, siehe z. B. DE 102 31 891 A1.

Erfindungsgemäß liegt die hergestellte Natriumdiformiat-Zubereitung in fester Form, insbesondere als Kristallisatpulver oder als Granulat oder Kompaktat vor. Je nach anwendungstechnischer Anforderung weisen die Pulver, Granulate bzw. Kompaktate eine mittlere Partikelgröße im Bereich von 1 µm bis 10000 µm, insbesondere von 10 µm bis 5000 µm und speziell von 100 µm bis 2500 µm auf.

Die erfindungsgemäß hergestellte feste Natriumdiformiat-Zubereitung bzw. diese enthaltende Formulierungen und Zusammensetzungen eignen sich zur Verwendung in Futtermitteln für Tiere (Tierfuttermittel), insbesondere als Zusatz zu Tierfutter in Form von Futtermitteladditiven (Futtermittelzusatzstoffe) und speziell als Zusatz zu Prämixen für Tierfuttermittel. Prämixe sind Mischungen, die in der Regel Mineralstoffe, Vitamine, Aminosäure, Spurenelemente sowie gegebenenfalls Enzyme enthalten. Tierfuttermittel und Futtermittelzusatzstoffe, die die erfindungsgemäß hergestellte feste Natriumdiformiat-Zubereitung enthalten, sind besonders geeignet für Monogastrier wie Schweine, speziell Ferkel, Zuchtsauen und Mastschweine, sowie Geflügel, speziell Broiler, Legehennen, Puten, Enten, Gänse, Wachteln, Fasane und Strauße.

In Abhängigkeit von den übrigen im Futtermittel oder Futtermitteladditiv enthaltenen Stoffen bzw. Zusatzstoffen kann der Gehalt der erfindungsgemäß hergestellten festen Natriumdiformiat-Zubereitung im Futtermittel bzw. Futtermitteladditiv stark variieren. Bei Futtermitteladditiven hängt der Gehalt außerdem von der Art der Formulierung ab, z. B. vom Zusatz von Hilfsstoffen wie Trockenmitteln, von einer eventuellen Beschichtung und vom Restfeuchtegehalt. Üblicherweise liegt der Gehalt an erfindungsgemäß hergestellter fester Natriumdiformiat-Zubereitung im Futtermitteladditiv z. B. im Bereich von 0,1 bis 99,5 Gew.-%, insbesondere von 0,5 bis 75 Gew.-% und speziell von 1 bis 50 Gew.-%, bezogen auf das Gesamttrockengewicht des Futtermitteladditivs. Die erfindungsgemäß hergestellte feste Natriumdiformiat-Zubereitung ist auch zur Verwendung in einem Prämix geeignet und kann hierbei in den üblichen Mengen eingesetzt, z. B. zugemischt, werden.

Insbesondere beim Einsatz in Tierfuttermittel und Futtermitteladditiven für Geflügel ist ein geringer Gehalt an Kaliumionen vorteilhaft, da Kalium in diesem Fall eine diurethische Wirkung entfalten kann. Der Einsatz der erfindungsgemäß hergestellten Natriumdiformiat-Zubereitung zu dem vorgenannten Zweck stellt somit eine saure Natrium- und Formiatquelle dar, ohne dass notwendigerweise der Anteil an Kaliumionen erhöht ist. So kann ein festes Futtermitteladditiv formuliert werden, welches die erfindungsgemäß hergestellte feste Natriumdiformiat-Zubereitung enthält und im Wesentlichen frei von Kaliumionen ist. Hierbei bedeutet im Wesentlichen frei von Kaliumionen, dass der Gehalt an Kaliumionen höchstens 1000 ppm und insbesondere höchstens 500 ppm, jeweils bezogen auf das Gewicht des Futtermitteladditivs, beträgt.

Tierfuttermittel werden so zusammengesetzt, dass der entsprechende Bedarf an Nährstoffen für die jeweilige Tierart optimal gedeckt wird. Im Allgemeinen werden pflanzliche Futtermittelkomponenten wie Mais-, Weizen- oder Gerstenschrot, Sojavollbohnenschrot, Sojaextraktionsschrot, Leinextraktionsschrot, Rapsextraktionsschrot, Grünmehl oder Erbsenschrot als Rohproteinquellen gewählt. Um einen entsprechenden Energiegehalt des Futtermittels zu gewährleisten, werden Sojaöl oder andere tierische oder pflanzliche Fette zugegeben. Da die pflanzlichen Proteinquellen einige essentielle Aminosäuren nur in unzureichender Menge beinhalten, werden Futtermittel häufig mit Aminosäuren angereichert. Hierbei handelt es sich vor allem um Lysin und Methionin. Um die Mineralstoff- und Vitaminversorgung der Nutztiere zu gewährleisten, werden außerdem Mineralstoffe und Vitamine zugesetzt. Die Art und Menge der zugesetzten Mineralstoffe und Vitamine hängt von der Tierspezies ab und ist dem Fachmann bekannt (s. z. B. Jeroch et al., Ernährung landwirtschaftlicher Nutztiere, Ulmer, UTB). Zur Deckung des Nährstoff- und Energiebedarfs können Alleinfutter verwendet werden, die alle Nährstoffe im bedarfsdeckenden Verhältnis zueinander enthalten. Es kann das einzige Futter der Tiere bilden. Alternativ kann zu einem Körnerfutter aus Getreide ein Ergänzungsfutter gegeben werden. Hierbei handelt es sich um eiweiß-, mineratstoff- und vitaminreiche Futtermischungen, die das Futter ergänzen.

Die erfindungsgemäß hergestellte feste Natriumdiformiat-Zubereitung eignet sich insbesondere als sogenannter Acidifier. Unter Acidifiern werden solche Stoffe verstanden, die den pH-Wert absenken. Der Ausdruck umfasst sowohl solche Stoffe, die den pH-Wert im Substrat (z. B. Tierfutter) absenken, als auch solche, die den pH-Wert im Magen-Darm Trakt des Tieres absenken.

Die erfindungsgemäß hergestellte feste Natriumdiformiat-Zubereitung eignet sich insbesondere als Mittel mit leistungs- und/oder wachstumsfördernder Wirkung. In einer bevorzugten Ausführungsform wird die feste Natriumdiformiat-Zubereitung als ein solches leistungs- und/oder wachstumsförderndes Mittel für Monogastrier, insbesondere für Schweine und/oder Geflügel eingesetzt.

Die erfindungsgemäß hergestellte feste Natriumdiformiat-Zubereitung eignet sich weiterhin als Konservierungsmittel, insbesondere als Konservierungsmittel für Grünfutter und/oder Tierfutter.

Die erfindungsgemäß hergestellte feste Natriumdiformiat-Zubereitung kann vorteilhafterweise bei der Herstellung von Silage eingesetzt werden. Sie beschleunigt die Milchsäuregärung bzw. verhindert ein Nachgären und hemmt die Entwicklung schädlicher Hefen, so dass sie als Silierungmittel (Silierhilfsmittel) verwendet werden kann.

Auch eine Verwendung der erfindungsgemäß hergestellten festen Natriumdiformiat-Zubereitung als Düngemittel ist möglich.

### Beschreibung der Figuren

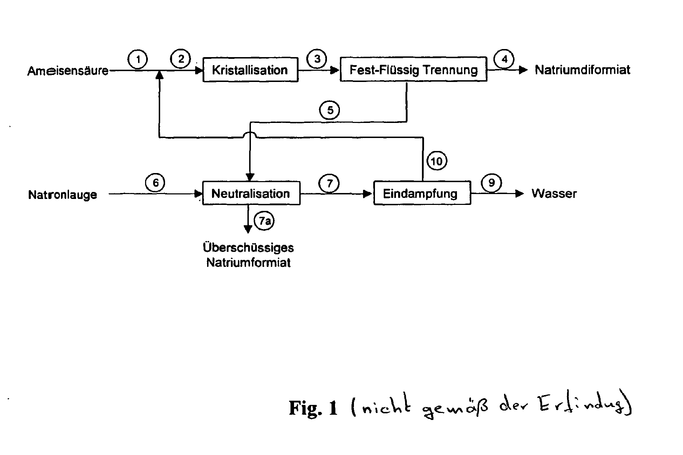

Fig. 1 zeigt eine schematische Darstellung einer Verfahrensvariante der EP 0 824 511 B1, bei der die Mutterlauge nach vollständiger Neutralisation der Kristallisationsstufe zurückgeführt wird. Die Fig. 1 entspricht im Wesentlichen der Fig. 2 der EP 0 824 511 B1, wobei letztere jedoch den ausgeschleusten Strom (7a) nicht zeigt. Natriumdiformiat kann bei dieser Verfahrensvariante nur erhalten werden, wenn in der Reaktionsmischung, aus der das Produkt auskristallisiert werden soll, die molaren Verhältnisse von Ameisensäure zu Natriumformiat bzw. zu Wasser entsprechend den für das erfindungsgemäße Verfahren gemachten Angaben eingestellt werden.

Im Einzelnen geht man bei dem in Fig. 1 dargestellten Verfahren so vor, dass man Ameisensäure als Strom (1) bereitstellt und mit einem Natriumformiat enthaltenden Strom (10) unter Erhalt eines die Reaktionsmischung darstellenden Stroms (2) zusammenführt. Die Reaktionsmischung wird als Strom (2) einer Kristallisationsstufe zugeführt und unter Erhalt einer festen Phase und einer Mutterlauge zur Kristallisation gebracht. Die feste Phase und die Mutterlauge werden in Form des Stroms (3) einer Trennungsstufe zugeführt, in der man die feste Phase von der Mutterlauge abtrennt, wobei man einen Natriumdiformiat enthaltenden Strom (4) und einen die Mutterlauge enthaltenden Strom (5) erhält. Den Strom (5) führt man mit einem Natronlauge-Strom (6) einer Neutralisationsstufe zu, wobei ein Natriumformiat enthaltendes Gemisch resultiert. Von diesem Gemisch entnimmt man einen Teil als Strom (7a). Den verbleibenden Teil führt man als Strom (7) einer Konzentrierungsstufe zu, in der man einen Teil des im Strom (7) enthaltenen Wassers als Strom (9) austrägt. Hierbei erhält man den Natriumformiat enthaltenden Strom (10), den man in den Schritt b) zurückführt.

Fig. 2 zeigt eine schematische Darstellung einer Verfahrensvariante des erfindungsgemäßen Verfahrens. Der Strom (5b) wird neutralisiert, durch Eindampfen aufkonzentriert und in die Kristallisationsstufe zurückgeführt.

Im Einzelnen geht man bei dem in Fig. 2 dargestellten Verfahren allgemein so vor, dass man einen Strom (1) der Ameisensäure mit einem Ameisensäuregehalt von wenigstens 74 Gew.-% bereitstellt. Den Strom (1) führt man mit zwei das Natriumformiat enthaltenden Strömen (5a) und (10) unter Erhalt eines Stroms (2) zusammen. Den Strom (2) führt man einer Kristallisationsstufe zu, wobei man, gegebenenfalls unter Temperaturerhöhung, die wässrige Lösung erhält, die ein molares Verhältnis von HCOOH : HCOONa von mehr als 1,5 : 1 und ein molares Verhältnis von HCOOH : H₂O von mindestens 1,1:1 aufweist. Die wässrige Lösung bringt man unter Erhalt der festen Phase und der Mutterlauge zur Kristallisation, z. B. mittels Eindampfen und/oder Temperaturerniedrigung. Die feste Phase und die Mutterlauge führt man in Form eines Stroms (3) einer Trennungsstufe zu, in der man die feste Phase von der Mutterlauge abtrennt. Den Natriumdiformiat enthaltenden Strom (4) schleust man aus. Den die Mutterlauge enthaltenden Strom (5) teilt man in zwei Teilströme (5a) und (5b) auf. Der Strom (5a) wird als Teilmenge (A) in die Kristallisationsstufe zurückgeführt. Den Strom (5b) führt man als Teilmenge (B) mit einem Natriumhydroxid- und/oder Natriumcarbonat-haltigen Strom (6) einer Neutralisationsstufe zu. Von dem bei der Neutralisation resultierenden Natriumformiat enthaltenden Gemisch entnimmt man gegebenenfalls einen Teil in Form des Stroms (7a). Den verbleibenden Teil führt man als Strom (7) einer Konzentrierungsstufe (Eindampfung) zu, in der man einen Teil des im Strom (7) enthaltenen Wassers als Strom (9) austrägt. Den hierbei erhaltenen Natriumformiat enthaltenden Strom (10) führt man in die Kristallisationsstufe zurück, der man auch die Ströme (1) und (5a) zuführt.

Fig. 3 zeigt eine schematische Darstellung einer Verfahrensvariante des erfindungsgemäßen Verfahrens. Der Strom (5b) wird neutralisiert und eingedampft. Der Wassergehalt des resultierenden Natriumformiat-haltigen Stroms (10) wird vor der Rückführung in die (erste) Kristallisationsstufe separat durch Kristallisation und Phasentrennung verringert.

Im Einzelnen geht man bei dem in Fig. 3 dargestellten Verfahren so vor, dass man einen Strom (1) der Ameisensäure mit einem Ameisensäuregehalt von wenigstens 74 Gew.-% bereitstellt. Den Strom (1) führt man mit zwei das Natriumformiat enthaltenden Strömen (5a) und (14) unter Erhalt eines Stroms (2) zusammen. Den Strom (2) führt man einer ersten Kristallisationsstufe zu, wobei man, gegebenenfalls unter Temperaturerhöhung, die wässrige Lösung erhält, die ein molares Verhältnis von HCOOH : Na[HCOO] von mehr als 1,5 : 1 und ein molares Verhältnis von HCOOH : H₂O von mindestens 1,1 : 1 aufweist. Die wässrige Lösung bringt man unter Erhalt der festen Phase und der Mutterlauge zur Kristallisation, z. B. mittels Eindampfen oder Temperaturerniedrigung. Die feste Phase und die Mutterlauge führt man in Form eines Stroms (3) einer ersten Trennungsstufe zu, in der man die feste Phase von der Mutterlauge abtrennt. Den Natriumdiformiat enthaltenden Strom (4) schleust man aus. Den die Mutterlauge enthaltenden Strom (5) teilt man in zwei Teilströme (5a) und (5b) auf. Der Strom (5a) wird als Teilmenge (A) in den Schritt b) zurückgeführt. Den Strom (5b) führt man als Teilmenge (B) mit einem Natriumhydroxid- und/oder Natriumcarbonat-haltigen Strom (6) einer Neutralisationsstufe zu. Von dem bei der Neutralisation resultierenden Natriumformiat enthaltenden Gemisch entnimmt man gegebenenfalls einen Teil (nicht gezeigt). Das Gemisch wird mit einem Natriumformiat-haltigen Strom (13a) unter Erhalt eines Stroms (8) zusammengeführt. Der Strom (8) wird einer Konzentrierungsstufe (Eindampfung) zugeführt, in der man einen Teil des im Strom (8) enthaltenen Wassers als Strom (9) austrägt. Der Wassergehalt des hierbei erhaltenen Natriumformiat enthaltenden Stroms (10) wird vor der Rückführung in den Schritt b) durch die nachfolgenden Schritte verringert. Den Strom (10) führt man mit einem Natriumformiat enthaltenden Strom (13b) unter Erhalt eines Stroms (11) zusammen. Den Strom (11) führt man einer zweiten Kristallisationsstufe zu, in der man den Strom (11) unter Erhalt einer zweiten festen Phase und einer zweiten Mutterlauge zur Kristallisation bringt, z.B. mittels Eindampfen oder Temperaturerniedrigung. Die zweite feste Phase und zweite Mutterlauge führt man in Form eines Stroms (12) einer zweiten Trennungsstufe zu, in der man die zweite feste Phase von der zweiten Mutterlauge abtrennt. Hierbei erhält man einen die zweite Mutterlauge enthaltenden Strom (13) und einen Natriumformiat enthaltenden Strom (14). Den Natriumformiat enthaltenden Strom (14) führt man in die erste Kristallisationsstufe zurück. Den die Mutterlauge enthaltenden Strom (13) teilt man in zwei Teilströme (13a) und (13b). Der Teilstrom (13a) wird mit dem Strom (7) unter Erhalt des Stroms (8) zusammengeführt. Den Strom (8) führt man der Konzentrierungsstufe (Eindampfung) zu. Der Teilstrom (13b) wird mit dem Strom (10) unter Erhalt des Stroms (11) zusammengeführt. Den Strom (11) führt man der zweiten Kristallisationsstufe zu. Es ist bei dieser Verfahrensvariante zusätzlich möglich, den Strom (13) teilweise zu entnehmen und auszuschleusen (nicht gezeigt).

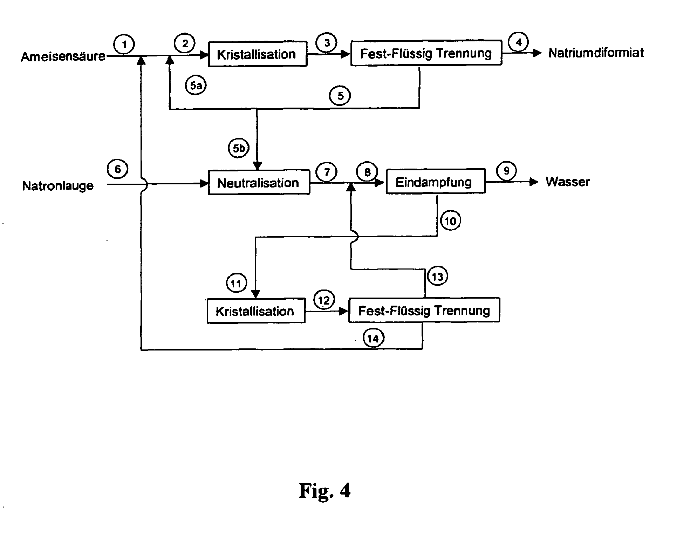

Fig. 4 zeigt eine schematische Darstellung einer Variante des in Fig. 3 gezeigten erfindungsgemäßen Verfahrens. Auch hierbei erhält man in einer zweiten Trennungsstufe einen die zweite Mutterlauge enthaltenden Strom (13) und einen Natriumformiat enthaltenden Strom (14). Den Natriumformiat enthaltenden Strom (14) führt man ebenfalls in die erste Kristallisationsstufe zurück. Den die Mutterlauge enthaltenden Strom (13) führt man mit dem Strom (7) unter Erhalt des Stroms (8) zusammen. Den Strom (8) führt man der Konzentrierungsstufe (Eindampfung) zu. Es ist bei dieser Verfahrensvariante zusätzlich möglich, den Strom (13) teilweise zu entnehmen und auszuschleusen (nicht gezeigt).

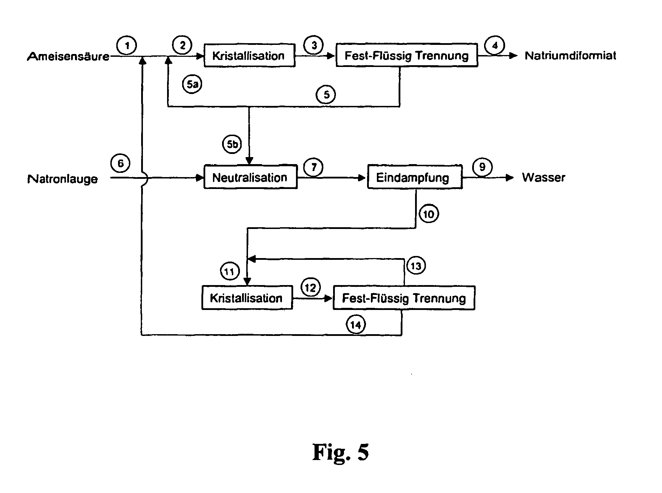

Fig. 5 zeigt eine schematische Darstellung einer weiteren Variante des in Fig. 3 gezeigten erfindungsgemäßen Verfahrens. Auch hierbei erhält man in einer zweiten Trennungsstufe einen die zweite Mutterlauge enthaltenden Strom (13) und einen Natriumformiat enthaltenden Strom (14). Den Natriumformiat enthaltenden Strom (14) führt man ebenfalls in die erste Kristallisationsstufe zurück. Den die Mutterlauge enthaltenden Strom (13) führt man mit dem Strom (10) unter Erhalt des Stroms (11) zusammen. Den Strom (11) führt man der zweiten Kristallisationsstufe zu. Es ist bei dieser Verfahrensvariante zusätzlich möglich, den Strom (13) teilweise zu entnehmen und auszuschleusen (nicht gezeigt).

Die nachfolgenden Beispiele dienen zur Veranschaulichung der Erfindung und sind in keiner Weise als einschränkend zu verstehen.

### Beispiele

### I. Herstellung von Natriumdiformiat-Zubereitungen ohne Rückführung von Mutterlauge (Vergleichsbeispiele)

Die Vergleichsbeispiele I.1 und I.2 wurden in einem 1 I Rührgefäß, das mit Heiz- und Kühlvorrichtung sowie einem Ablauf ausgestattet war, durchgeführt. Die molaren Verhältnisse der Komponenten in der zu kristallisierenden wässrigen Lösung entsprechen jeweils den beim erfindungsgemäßen Verfahren einzustellenden Werten.

### Vergleichsbeispiel I.1 (gemäß DE 102005017089.7)

650 g einer 94 gew.-%igen wässrigen Ameisensäurelösung wurden vorgelegt und unter Rühren auf 55 °C erhitzt. Das Rühren wurde während der gesamten Versuchsdauer fortgesetzt. Es wurden 350 g festes Natriumformiat (Reinheit > 97 %) in der Ameisensäurelösung gelöst, wobei eine klare Lösung erhalten wurde. Anschließend wurde die Lösung langsam abgekühlt. Nach etwa 4 Stunden wurde eine Temperatur von etwa 12 °C erreicht, bei der ein plötzlicher Niederschlag beobachtet wurde. Die Suspension wurde auf etwa 35 °C erhitzt, bis nur noch eine leichte Trübung beobachtet wurde. Die Suspension wurde dann über einen Zeitraum von etwa 6 h auf 20 °C abgekühlt und aus dem Rührgefäß abgelassen. Die Mutterlauge wurde mittels einer Nutsche von den Kristallen abgetrennt. Die Ausbeute an feuchter Natriumdiformiat-Zubereitung betrug etwa 125 g. Nach der Trocknung im Vakuumtrockenschrank bei einer Temperatur von 35 °C wurde der restliche Wassergehalt im Produkt zu ca. 0,1 Gew.-%, bezogen auf das Gesamttrockengewicht von etwa 122 g, bestimmt. Der Gehalt an Ameisensäure im trockenen Produkt lag bei 40,3 Gew.-%, bezogen auf das Gesamttrockengewicht.

### Vergleichsbeispiel 1.2 (gemäß DE 102005017089.7)

650 g einer 80 gew.-%igen wässrigen Ameisensäurelösung wurden vorgelegt und unter Rühren auf 55 °C erhitzt. Unter fortgesetztem Rühren wurden 430 g festes Natriumformiat (Reinheit > 97 %) in der Ameisensäurelösung gelöst, wobei eine klare Lösung erhalten wurde. Anschließend wurde die Lösung langsam abgekühlt. Nach etwa 5 Stunden wurde eine Temperatur von etwa 24 °C erreicht, bei der ein plötzlicher Niederschlag beobachtet wurde. Die Suspension wurde unter Rühren auf etwa 35 °C erhitzt, bis nur noch eine leichte Trübung beobachtet wurde. Die Suspension wurde dann unter Rühren über einen Zeitraum von etwa 6 h auf 15 °C abgekühlt und aus dem Rührgefäß abgelassen. Die Mutterlauge wurde mittels einer Nutsche von den Kristallen abgetrennt. Die Ausbeute an feuchter Natriumdiformiat-Zubereitung betrug etwa 280 g Nach der Trocknung im Vakuumtrockenschrank bei einer Temperatur von 35 °C wurde der restliche Wassergehalt im Produkt zu ca. 0,15 Gew.-%, bezogen, auf das Gesamttrockengewicht von 270 g, bestimmt. Der Gehalt an Ameisensäure im trockenen Produkt lag bei 40,1 Gew.-%, bezogen auf das Gesamttrockengewicht.

### Vergleichsbeispiel I.3 (analog Beispiel 2 der DE 424017)

Die molaren Verhältnisse der Komponenten in der zu kristallisierenden wässrigen Lösung entsprechen nicht den beim erfindungsgemäßen Verfahren einzustellenden Werten.

476 g einer 80 gew.-%igen wässrigen Ameisensäurelösung wurden vorgelegt. Unter Rühren wurden 524 g festes Natriumformiat zugegeben. Zur vollständigen Lösung wurde auf eine Temperatur von 120 °C erhitzt. Anschließend wurde die Lösung langsam abgekühlt. Ab etwa 112 °C setzte Kristallisation ein. Es wurde mit einer Rate von etwa 0,7 K/min weiter bis auf 25 °C abgekühlt. Die Suspension wurde dann 24 h unter leichtem Rühren stehen gelassen. Danach wurden die gebildeten Kristalle von der Mutterlauge abgetrennt. Die Ausbeute an feuchtem Produkt betrug etwa 370 g. Der Gehalt an Ameisensäure lag bei etwa 21,8 Gew.-%, bezogen auf das Gesamtgewicht des feuchten Produkts.

### II. Herstellung von Natriumdiformiat-Zubereitungen mit Rückführung von Mutterlauge

### Vergleichsbeispiel II.1 (Stromführung analog EP 0 824 511 B1)

Es wird Bezug genommen auf die beigefügte Fig. 1, die eine schematische Darstellung einer Verfahrensvariante der EP 0 824 511 B1 zeigt, bei der die Mutterlauge nach vollständiger Neutralisation in die Kristallisationsstufe zurückgeführt wird. Die Fig. 1 entspricht im Wesentlichen der Fig. 2 der EP 0 824 511 B1, wobei letztere jedoch den ausgeschleusten Strom (7a) nicht zeigt.

In den nachfolgenden Tabellen 1 a und 1 b ist beispielhaft eine Bilanz der Stoffflüsse angegeben, die sich unter Zugrundelegung der in der EP 0 824 511 B1 gemachten Angaben zur Stromführung gemäß der Fig. 2 der EP 0 824 511 B1 erstellen lässt. Hierbei wird als Strom (1) eine 85 gew.-%ige Ameisensäurelösung und als Strom (6) eine 50 gew.-%ige wässrige Natronlauge zugeführt, als Strom (10) wird eine 80 gew.-%ige Natriumformiatlösung in die Kristallisationsstufe (Kristallisation) zurückgeführt.

In diesem Zusammenhang sei jedoch ausdrücklich darauf hingewiesen, dass der EP 0 824 511 B1 außer den vorgenannten Stoffflüssen keine weiteren konkreten Angaben zu den einzustellenden Stoffflüssen zu entnehmen sind. Insbesondere entspricht das bei den hier dargestellten Beispielen II.1 und II.2 eingestellte molare Verhältnis von Ameisensäure zu Natriumformiat in dem zu kristallisierenden Strom (2) nicht den gemäß der EP 0 824 511 B1 als bevorzugt angegebenen Molverhältnissen, sondern wurde entsprechend dem für das erfindungsgemäße Verfahren einzuhaltenden Bereich eingestellt, da nur so sichergestellt ist, dass der erhaltene Strom (4) tatsächlich Natriumdiformiat in möglichst reiner Form enthält.

**Tabelle 1a**

| | | 1 | 2 | 3 | 4 | 5 | 6 7 | 7 | 7a | 9 | 10 |
|---|---|---|---|---|---|---|---|---|---|---|---|
| t/h | NaFo*AS (s) | | | 1 | 1 | | | | | | |
| | NaFo | | 1,41 | 0,81 | 0,03 | 0,78 | | 1,38 | 1,56 | | 1,38 |
| | AS | 1,86 | 1,92 | 1,52 | 0,06 | 1,46 | | | | | |
| | H2O | 0,33 | 0,67 | 0,67 | 0,02 | 0,65 | 1,27 | 1,17 | 1,32 | 0,82 | 0,34 |
| | NaOH | | | | | | 1,27 | | | | |
| | Gesamt | 2,19 | 4,00 | 4,00 | 1,11 | 2,89 | 2,54 | 2,54 | 2,88 | | 1,72 |
| Lösung Gew-% | NaFo | | 35,17 | 27,01 | | 27,01 | | 54,14 | 54,14 | | 80,00 |
| | AS | 85,00 | 48,00 | 50,55 | | 50,55 | | | | | |
| | H2O | 15,00 | 16,83 | 22,44 | | 22,44 | 50,00 | 45,86 | 45,86 | | 20,00 |
| | NaOH | | | | | | 50,00 | | | | |

**Tabelle 1b**

| | | 1 | 2 | 3 | 4 | 5 | 6 | 7 | 7a | 9 | 10 |
|---|---|---|---|---|---|---|---|---|---|---|---|
| kmol/h | NaFo*AS (s) | | | 1 | 1 | | | | | | |
| | NaFo | | 2,36 | 1,36 | 0,05 | 1,31 | | 2,31 | 2,62 | | 2,31 |
| | AS | 4,62 | 4,76 | 3,76 | 0,14 | 3,62 | | | | | |
| | H2O | 2,08 | 4,26 | 4,26 | 0,16 | 4,10 | 8,04 | 7,38 | 8,38 | 5,21 | 2,18 |
| | NaOH | | | | | | 3,62 | | | | |
| | Gesamt | 6,70 | 11,38 | 10,38 | 1,35 | 9,03 | 11,66 | 9,69 | 10,99 | 5,21 | 4,49 |
| Lösung Mol-% | NaFo | | 20,73 | 14,48 | 14,48 | 14,48 | | 23,81 | 23,81 | | 51,43 |
| | AS | 68,92 | 41,81 | 40,07 | 40,07 | 40,07 | | | | | |
| | H2O | 31,08 | 37,46 | 45,45 | 45,45 | 45,45 | 68,97 | 76,19 | 76,19 | 100,0 | 48,57 |
| | NaOH | | | | | | 31,03 | | | | |

In den Tabellen 1a und 1 b steht NaFo*AS (s) für festes Natriumdiformiat, NaFo für Natriumformiat, AS für Ameisensäure, H2O für Wasser, NaOH für Natriumhydroxid; die Ziffern 1 bis 10 in der ersten Zeile jeder Tabelle bezeichnen den mit der jeweiligen Nummer gekennzeichneten Strom in der Fig. 1; Lösung Gew.-% bezeichnet die Gewichtsteile der Komponenten im jeweiligen flüssigen Teil des Stroms (d. h. ohne Feststoffanteile), Lösung Mol-% die entsprechenden molaren Anteile.

Die Berechnung der obigen Bilanz zeigt, dass die hier verwendeten Gewichtsverhältnisse der Komponenten in den Strömen (1) und (10) nur eingestellt werden können, wenn ein erheblicher Teil, nämlich mehr als 50 Gew.-%, der in der Neutralisationsstufe angefallenen Natriumformiatlösung als Strom (7a) ausgeschleust wird. Darüber hinaus resultiert bei der Zusammenführung der Ströme (1) und (10) ein Strom (2), der einen Wassergehalt von mehr als 16 Gew.-% aufweist und somit eine Kristallisationstemperatur von weniger als 20 °C besitzt.

### Vergleichsbeispiel II.2 (analog EP 0 824 511 B1)

Für das Vergleichsbeispiel II.2 wird ebenfalls auf die beigefügte Fig. 1 Bezug genommen. Als Strom (1) wird eine 94 gew.-%ige Ameisensäurelösung und als Strom (6) eine 50 gew.-%ige wässrige Natronlauge zugeführt, als Strom (10) wird eine 80 gew.-%ige Natriumformiatlösung in die Kristallisationsstufe (Kristallisation) zurückgeführt. Im Übrigen wird entsprechend Vergleichsbeispiel II.1 verfahren. Die nachfolgenden Tabellen 2a und 2b geben die hierbei berechnete Bilanz der Stoffflüsse wieder.

**Tabelle 2a**

| | | 1 | 2 | 3 | 4 | 5 | 6 | 7 | 7a | 9 | 10 |
|---|---|---|---|---|---|---|---|---|---|---|---|
| t/h | NaFo*AS (s) | | | 1 | 1 | | | | | | |
| | NaFo | | 1,31 | 0,72 | 0,03 | 0,68 | | 1,28 | 1,09 | | 1,28 |
| | AS | 1,54 | 1,60 | 1,20 | 0,06 | 1,14 | | | | | |
| | H2O | 0,10 | 0,42 | 0,42 | 0,02 | 0,40 | 0,99 | 0,99 | 0,84 | 0,67 | 0,32 |
| | NaOH | | | | | | 0,99 | | | | |
| | Gesamt | 1,64 | 3,33 | 3,33 | 1,11 | 2,22 | 1,98 | 2,27 | 1,93 | | 1,60 |
| Lösung Gew-% | NaFo | | 39,44 | 30,78 | | 30,78 | | 56,34 | 56,34 | | 80,00 |
| | AS | 94,00 | 48,00 | 51,28 | | 51,28 | | | | | |
| | H2O | 6,00 | 12,56 | 17,94 | | 17,94 | 50,00 | 43,66 | 43,66 | | 20,00 |
| | NaOH | | | | | | 50,00 | | | | |

**Tabelle 2b**

| | | 1 | 2 | 3 | 4 | 5 | 6 | 7 | 7a | 9 | 10 |
|---|---|---|---|---|---|---|---|---|---|---|---|
| | NaFo*AS (s) | | | 1 | 1 | | | | | | |
| kmol/h | NaFo | | 2,20 | 1,20 | 0,06 | 1,15 | | 2,15 | 1,82 | | 2,15 |
| | AS | 3,82 | 3,96 | 2,96 | 0,14 | 2,82 | | | | | |
| | H2O | 0,62 | 2,65 | 2,65 | 0,13 | 2,52 | 6,27 | 6,28 | 5,34 | 4,26 | 2,03 |
| | NaOH | | | | | | 2,82 | | | | |
| | Gesamt | 4,45 | 8,82 | 7,82 | 1,32 | 6,50 | 9,10 | 8,43 | 7,16 | 4,26 | 4,17 |
| Lösung Mol-% | NaFo | | 24,99 | 17,65 | 17,65 | 17,65 | | 25,46 | 25,46 | | 51,43 |
| | AS | 85,98 | 44,95 | 43,47 | 43,47 | 43,47 | | | | | |
| | H2O | 14,02 | 30,06 | 38,87 | 38,87 | 38,87 | 68,97 | 74,54 | 74,54 | 100,0 | 48,57 |
| | NaOH | | | | | | 31,03 | | | | |

Die Berechnung der obigen Bilanz zeigt, dass die hier verwendeten Gewichtsverhältnisse der Komponenten in den Strömen (1) und (10) nur eingestellt werden können, wenn ein erheblicher Teil, nämlich etwa 46 Gew.-%, der in der Neutralisationsstufe angefallenen Natriumformiatlösung als Strom (7a) ausgeschleust wird.

### Beispiel II.1

Es wird Bezug genommen auf die beigefügte Fig. 2, die eine schematische Darstellung einer Verfahrensvariante des erfindungsgemäßen Verfahrens zeigt, bei der die Mutterlauge in zwei Teilströme (5a) und (5b) aufgeteilt wird. Der Strom (5a) wird direkt in die Kristallisationsstufe zurückgeführt, der Strom (5b) erst nach vollständiger Neutralisation mit dem Strom (6).

Die nachfolgenden Tabellen 3a und 3b geben die hierbei berechnete Bilanz der Stoffflüsse wieder. Als Strom (1) wird eine 94 gew.-%ige Ameisensäurelösung und als Strom (6) eine 50 gew.-%ige wässrige Natronlauge zugeführt. Die Aufteilung des Stroms (5) in die Teilströme (5a) und (5b) ist so gewählt, dass das Gewichtsverhältnis von Strom (5a) zu Strom (5b) etwa 2,4 : 1 beträgt und das molare Verhältnis von Ameisensäure im Strom (5b) zur Gesamtmenge von Natriumdiformiat und Natriumformiat im Strom (4) möglichst 1 : 1 beträgt. Hierbei wird als Strom (10) eine 85 gew.-%ige Lösung oder Suspension von Natriumformiat in die Kristallisationsstufe (Kristallisation) zurückgeführt.

**Tabelle 3a**

| | | 1 | 2 | 3 | 4 | 5 | 5a | 5b | 6 | 7 | 7a | 9 | 10 |
|---|---|---|---|---|---|---|---|---|---|---|---|---|---|
| | NaFo*AS (s) | | | 1,00 | 1,00 | | | | | | | | |
| t/h | NaFo | | 1,42 | 0,83 | 0,03 | 0,80 | 0,56 | 0,23 | | 0,86 | | | 0,86 |
| | AS | 0,88 | 1,92 | 1,52 | 0,06 | 1,46 | 1,04 | 0,42 | | | | | |
| | H2O | 0,06 | 0,66 | 0,66 | 0,02 | 0,63 | 0,45 | 0,18 | 0,37 | 0,72 | | 0,57 | 0,15 |
| | NaOH | | | | | | | | 0,37 | | | | |
| | Gesamt | 0,94 | 4,00 | 4,00 | 1,11 | 2,89 | 2,05 | 0,84 | 0,74 | 1,58 | 0,00 | 0,57 | 1,01 |
| Lösung Gew-% | NaFo | | 35,59 | 27,57 | 27,57 | 27,57 | 27,57 | 27,57 | | 54,44 | | | 85,00 |
| | AS | 94,00 | 48,00 | 50,55 | 50,55 | 50,55 | 50,55 | 50,55 | | | | | |
| | H2O | 6,00 | 16,41 | 21,88 | 21,88 | 21,88 | 21,88 | 21,88 | 50,00 | 45,56 | | 100,0 | 15,00 |
| | NaOH | | | | | | | | 50,00 | | | | |

**Tabelle 3b**

| | | 1 | 2 | 3 | 4 | 5 | 5a | 5b | 6 | 7 | 7a | 9 | 10 |
|---|---|---|---|---|---|---|---|---|---|---|---|---|---|
| | NaFo*AS (s) | | | 1 | 1 | | | | | | | | |
| kmol/h | NaFo | | 2,39 | 1,39 | 0,05 | 1,33 | 0,95 | 0,39 | | 1,44 | | | 1,44 |
| | AS | 2,19 | 4,76 | 3,76 | 0,14 | 3,62 | 2,57 | 1,05 | | | | | |
| | H2O | 0,36 | 4,15 | 4,15 | 0,15 | 4,00 | 2,84 | 1,16 | 2,34 | 4,55 | | 3,59 | 0,96 |
| | NaOH | | | | | | | | 1,05 | | | | |
| | Gesamt | 2,55 | 11,30 | 10,30 | 1,34 | 8,95 | 6,35 | 2,60 | 3,39 | 5,99 | 0,00 | 3,59 | 2,40 |
| Lösung Mol-% | NaFo | | 21,12 | 14,91 | 14,91 | 14,91 | 14,91 | 14,91 | | 24,03 | | | 60,00 |
| | AS | 85,98 | 42,10 | 40,40 | 40,40 | 40,40 | 40,40 | 40,40 | | | | | |
| | H2O | 14,02 | 36,78 | 44,69 | 44,69 | 44,69 | 44,69 | 44,69 | 68,97 | 75,97 | | 100,0 | 40,00 |
| | NaOH | | | | | | | | 31,03 | | | | |

Die Berechnung der obigen Bilanz zeigt, dass die hier verwendete Aufteilung des Stroms (5) in die Ströme (5a) und (5b) dazu führt, dass in der Neutralisationsstufe kein überschüssiges Natriumformiat anfällt, so dass eine etwaige Ausschleusung über den Strom (7a) entfallen kann.

### Beispiel II.2

Es wird Bezug genommen auf die beigefügte Fig. 3, die eine schematische Darstellung einer Verfahrensvariante des erfindungsgemäßen Verfahrens zeigt, bei der die Mutterlauge in zwei Teilströme (5a) und (5b) aufgeteilt wird. Der Strom (5a) wird direkt in die (erste) Kristallisationsstufe zurückgeführt. Der Strom (5b) wird mit dem Strom (6) vollständig neutralisiert und eingedampft. Der resultierende Natriumformiat-haltige Strom (10) wird (in der zweiten Kristallisationsstufe) zur Kristallisation gebracht; die hierbei erhaltene Flüssigphase wird als Strom (13) zusammen mit Strom (7) der Verdampfungsstufe zugeführt, die feste Natriumformiat-haltige Phase wird als Strom (14) zusammen mit den Strömen (1) und (5a) der (ersten) Kristallisationsstufe zugeführt.

Die nachfolgenden Tabellen 4a und 4b geben die hierbei berechnete Bilanz der Stoffflüsse wieder. Als Strom (1) wird eine 94 gew.-%ige Ameisensäurelösung und als Strom (6) eine 50 gew.-%ige wässrige Natronlauge zugeführt. Die Aufteilung des Stroms (5) in die Teilströme (5a) und (5b) ist so gewählt, dass das Gewichtsverhältnis von Strom (5a) zu Strom (5b) etwa 2 : 1 beträgt und das molare Verhältnis von Ameisensäure im Strom (5b) zur Gesamtmenge von Natriumdiformiat und Natriumformiat im Strom (4) möglichst 1 : 1 beträgt. Hierbei wird Natriumformiat in Form des Stroms (14) im Wesentlichen als Feststoff mit einem geringen Anteil an Restfeuchte in die (erste) Kristallisationsstufe zurückgeführt.

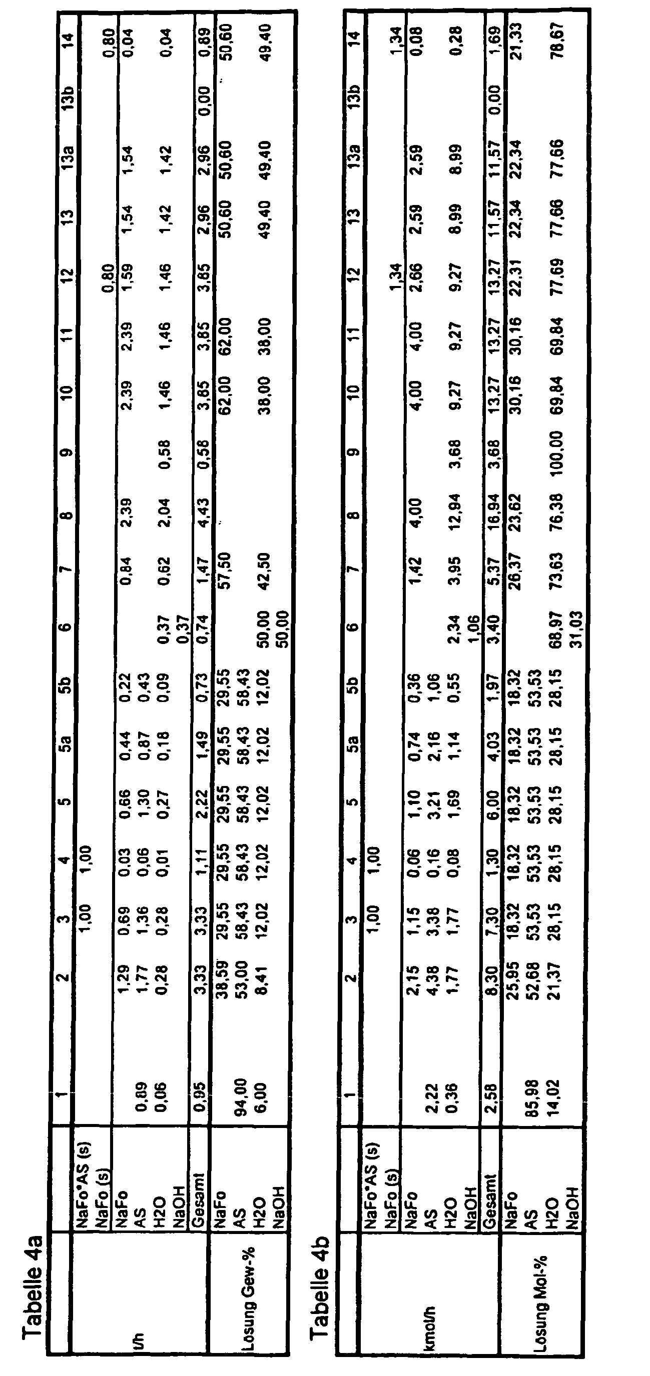

Die Berechnung der obigen Bilanz zeigt, dass die hier verwendete Aufteilung des Stroms (5) in die Ströme (5a) und (5b) dazu führt, dass in der Neutralisationsstufe kein überschüssiges Natriumformiat anfällt, so dass eine etwaige Ausschleusung entfallen kann. Darüber hinaus kann der Wassergehalt des in die erste Kristallisationsstufe zurückgeführten Natriumformiat-haltigen Stroms (14) hierbei auf einen sehr geringen Wert eingestellt werden.

## Patentansprüche

1. Verfahren zur Herstellung einer festen Natriumdiformiat-Zubereitung mit einem Gehalt an Ameisensäure von mindestens 35 Gew.-%, bezogen auf das Gesamtgewicht der Natriumdiformiat-Zubereitung, bei dem man bei erhöhter Temperatur aus Natriumformiat und wenigstens 74 gew.-%iger Ameisensäure eine wässrige Lösung herstellt, die ein molares Verhältnis von HCOOH : HCOONa von mehr als 1,5 : 1 und ein molares Verhältnis von HCOOH : H₂O von mindestens 1,1 : 1 aufweist, die wässrige Lösung zur Kristallisation bringt und die feste Phase von der Mutterlauge abtrennt, wobei man
(i) eine Teilmenge (A) der Mutterlauge bei der Herstellung der wässrigen Lösung einsetzt und
(ii) eine Teilmenge (B) der Mutterlauge mit einer Natrium-haltigen Base versetzt und das hierbei resultierende Natriumformiat enthaltende Gemisch, gegebenenfalls nach Ausschleusung eines Teils desselben und gegebenenfalls nach Konzentrierung desselben, ebenfalls bei der Herstellung der wässrigen Lösung einsetzt;
und wobei sich die Teilmengen (A) und (B) der Mutterlauge zu 100 Gew.-% ergänzen.

2. Verfahren nach Anspruch 1, wobei die Herstellung der wässrigen Lösung bei einer Temperatur von nicht mehr als 100 °C erfolgt.

3. Verfahren nach einem der Ansprüche 1 oder 2, wobei das Gewichtsverhältnis von Teilmenge (A) zu Teilmenge (B) der Mutterlauge im Bereich von 20:1 bis 1:10 liegt.

4. Verfahren nach einem der vorherigen Ansprüche, wobei man das Mengenverhältnis von Teilmenge (A) zu Teilmenge (B) der Mutterlauge so einstellt, dass das molare Verhältnis von HCOOH in der Teilmenge (B) der Mutterlauge zu der Gesamtstoffmenge des in der erhaltenen festen Phase enthaltenen Natriumdiformiats und gegebenenfalls enthaltenen Natriumformiats, vor einer gegebenenfalls anschließenden Trocknung der festen Phase höchstens 1,2 : 1 beträgt.

5. Verfahren nach einem der vorherigen Ansprüche, wobei die Natrium-haltige Base in Schritt (ii) unter Natriumhydroxid, Natriumcarbonat, Natriumhydrogencarbonat, Natrium-C₁-C₆-alkanolaten und Mischungen davon ausgewählt ist.

6. Verfahren nach einem der vorherigen Ansprüche, wobei man in Schritt (ii) die Teilmenge (B) der Mutterlauge im Wesentlichen vollständig neutralisiert.

7. Verfahren nach einem der vorherigen Ansprüche, wobei man in Schritt (ii) höchstens 20 Gew.-% des Natriumformiat enthaltenden Gemischs, bezogen auf das Gesamtgewicht des Natriumformiat enthaltenden Gemischs, entnimmt.

8. Verfahren nach einem der vorherigen Ansprüche, wobei man von dem in Schritt (ii) erhaltenen Natriumformiat enthaltenden Gemisch einen Teil entnimmt und ausschleust, wobei die im verbleibenden Teil des Gemischs enthaltene Menge Natriumformiat zusammen mit der in der Teilmenge (A) der Mutterlauge enthaltenen Menge Natriumformiat die zur Herstellung der wässrigen Lösung eingesetzte Gesamtmenge Natriumformiat ergibt.

9. Verfahren nach einem der vorherigen Ansprüche, wobei man den Wassergehalt des Natriumformiat enthaltenden Gemischs aus Schritt (ii) vor dessen Verwendung bei der Herstellung der wässrigen Lösung auf höchstens 20 Gew.-%, bezogen auf das Gesamtgewicht des Gemischs, verringert.

10. Verfahren nach Anspruch 9, wobei man den Wassergehalt mittels einer Verdampfungsstufe verringert oder mittels einer zweiten Kristallisationsstufe, in der man eine zweite feste Phase und eine zweite Mutterlauge erhält, und einer zweiten Konzentrierungsstufe, in der man die zweite feste Phase von der zweiten Mutterlauge abtrennt, verringert.

11. Verfahren nach einem der Ansprüche 1 bis 9, bei dem man
a) einen Strom (1) der Ameisensäure mit einem Ameisensäuregehalt von wenigstens 74 Gew.-% bereitstellt;
b) den Strom (1) aus Schritt a) mit zwei das Natriumformiat enthaltenden Strömen (5a) und (10) einer Kristallisationsstufe zuführt, wobei man, gegebenenfalls unter Temperaturerhöhung, die wässrige Lösung, die ein molares Verhältnis von HCOOH : Na[HCOO] von mehr als 1,5 : 1 und ein molares Verhältnis von HCOOH : H₂O von mindestens 1,1 : 1 aufweist, erhält;
c) in der Kristallisationsstufe die wässrige Lösung aus Schritt b) unter Erhalt eines die feste Phase und die Mutterlauge aufweisenden Stroms (3) zur Kristallisation bringt;
d) den Strom (3) aus Schritt c) einer Trennungsstufe zuführt, in der man die feste Phase von der Mutterlauge abtrennt, wobei man einen das Natriumdiformiat enthaltenden Strom (4) und einen die Mutterlauge enthaltenden Strom (5) erhält;
e) den Strom (5) aus Schritt d) in zwei Teilströme (5a) und (5b) aufteilt;
f) den Strom (5a) aus Schritt e) als Teilmenge (A) in den Schritt b) zurückführt;
g) den Strom (5b) aus Schritt e) als Teilmenge (B) mit einem die Natrium-haltige Base enthaltenden Strom (6) einer Neutralisationsstufe zuführt, wobei das Natriumformiat enthaltende Gemisch resultiert; und
h) das Natriumformiat enthaltende Gemisch aus Schritt g), gegebenenfalls nach Entnahme eines Teils desselben in Form des Stroms (7a), als Strom (7) einer Konzentrierungsstufe zuführt, in der man einen Teil des im Strom (7) enthaltenen Wassers als Strom (9) austrägt, wobei man den Natriumformiat enthaltenden Strom (10) erhält, den man in den Schritt b) zurückführt.

12. Verfahren nach Anspruch 11, wobei im Schritt d) der Strom (5) im Wesentlichen Ameisensäure im Bereich von 35 bis 80 Gew.-%, Natriumformiat im Bereich von 20 bis 45 Gew.-% und Wasser im Bereich von 0 bis 30 Gew.-%, jeweils bezogen auf das Gesamtgewicht des Stroms (5), enthält.

13. Verfahren nach einem der Ansprüche 11 oder 12, wobei man im Schritt g) als Strom (6) eine wässrige Natronlauge mit einem Gehalt an NaOH im Bereich von 10 bis 60 Gew.-%, bezogen auf das Gesamtgewicht der wässrigen Natronlauge, einsetzt.

14. Verfahren nach einem der Ansprüche 11 bis 13, wobei der Strom (10) aus Schritt h) im Wesentlichen Natriumformiat im Bereich von 50 bis 100 Gew.-% und Wasser im Bereich von 0 bis 50 Gew.-%, jeweils bezogen auf das Gesamtgewicht des Stroms (10), enthält.

15. Verfahren nach einem der Ansprüche 11 bis 14, bei dem man zusätzlich
k) den Natriumformiat enthaltenden Strom (10) aus Schritt h) vor der Rückführung in den Schritt b) einer zweiten Kristallisationsstufe zuführt und hierin unter Erhalt einer zweiten festen Phase und einer zweiten Mutterlauge zur Kristallisation bringt;
l) die aus Schritt k) erhaltene zweite feste Phase und zweite Mutterlauge in Form eines Stroms (12) einer Trennungsstufe zuführt, in der man die zweite feste Phase von der zweiten Mutterlauge abtrennt, wobei man einen die zweite Mutterlauge enthaltenden Strom (13) und einen Natriumformiat enthaltenden Strom (14) erhält;
m) den Natriumformiat enthaltenden Strom (14) aus Schritt I) in den Schritt b) zurückführt und hierin als Strom (10) verwendet; und
n) den die Mutterlauge enthaltenden Strom (13) aus Schritt I)
n1) in den Schritt h) zurückführt und hierin mit dem Strom (7) der Konzentrierungsstufe des Schritts h) zuführt;
n2) in den Schritt k) zurückführt und hierin mit dem Strom (10) der zweiten Kristallisationsstufe zuführt;
n3) in die Teilströme (13a) und (13b) aufteilt, den Teilstrom (13a) in den Schritt h) zurückführt und hierin mit dem Strom (7) der Konzentrierungsstufe des Schritts h) zuführt und den Teilstrom (13b) in den Schritt k) zurückführt und hierin mit dem Strom (10) der zweiten Kristallisationsstufe zuführt; und/oder
n4) teilweise entnimmt und ausschleust.

16. Verfahren nach Anspruch 15, wobei der die Mutterlauge enthaltenden Strom (13) aus Schritt I) im Wesentlichen Wasser im Bereich von 20 bis 50 Gew.-% und Natriumformiat im Bereich von 50 bis 80 Gew.-%, jeweils bezogen auf das Gesamtgewicht des Stroms (13), enthält.

17. Verfahren nach einem der Ansprüche 15 oder 16, wobei man in Schritt n4) höchstens 30 Gew.-% des Stroms (13), bezogen auf das Gesamtgewicht des Stroms (13), entnimmt und ausschleust.

18. Verfahren nach einem der Ansprüche 15 bis 17, wobei man den die Mutterlauge enthaltenden Strom (13) aus Schritt I) gemäß Schritt n1) in den Schritt h) zurückführt und hierin mit dem Strom (7) der Konzentrierungsstufe zuführt.

19. Verfahren nach einem der Ansprüche 15 bis 18, wobei der Natriumformiat enthaltende Strom (14) aus Schritt I) im Wesentlichen Natriumformiat im Bereich von 75 bis 100 Gew.-% und Wasser im Bereich von 0 bis 25 Gew.-%, jeweils bezogen auf das Gesamtgewicht des Stroms (14), enthält.

20. Verfahren nach einem der vorherigen Ansprüche, wobei die feste Natriumdiformiat-Zubereitung einen Gehalt an Ameisensäure im Bereich von 38 bis 41 Gew.-%, bezogen auf das Gesamtgewicht der Natriumdiformiat-Zubereitung, aufweist.

21. Verfahren nach einem der vorherigen Ansprüche, wobei die feste Natriumdiformiat-Zubereitung einen Wassergehalt von nicht mehr als 0,5 Gew.-%, bezogen auf das Gesamtgewicht der Zubereitung, aufweist.

## Claims

1. A process for producing a solid sodium diformate preparation having a formic acid content of at least 35% by weight, based on the total weight of the sodium diformate preparation, in which, at elevated temperature from sodium formate and at least 74% strength by weight formic acid, an aqueous solution is produced which has a molar ratio of HCOOH:HCOONa of greater than 1.5:1 and a molar ratio of HCOOH:H₂O of at least 1.1:1, the aqueous solution is brought to crystallization, and the solid phase is separated off from the mother liquor,
(i) a subquantity (A) of the mother liquor being used in the production of the aqueous solution and
(ii) a subquantity (B) of the mother liquor being admixed with a sodium-containing base and the resultant mixture comprising sodium formate, optionally after ejecting a part of same, and optionally after concentrating same, likewise being used in the production of the aqueous solution;
and the subquantities (A) and (B) of the mother liquor totaling 100% by weight.

2. The process according to claim 1, wherein the aqueous solution is produced at a temperature of not above 100°C.

3. The process according to one of claims 1 or 2, wherein the weight ratio of subquantity (A) to subquantity (B) of the mother liquor is in the range form 20:1 to 1:10.

4. The process according to one of the preceding claims, wherein the ratio of subquantity (A) to subquantity (B) of the mother liquor is set in such a manner that the molar ratio of HCOOH in the subquantity (B) of the mother liquor to the total amount of substance of the sodium diformate present in the resultant solid phase, and optionally sodium formate present, before an optional subsequent drying of the solid phase, is at most 1.2:1.

5. The process according to one of the preceding claims, wherein the base comprising sodium in step (ii) is selected from sodium hydroxide, sodium carbonate, sodium hydrogencarbonate, sodium C₁-C₆-alkanolates and mixtures thereof.

6. The process according to one of the preceding claims, wherein, in step (ii), the subquantity (B) of the mother liquor is essentially completely neutralized.

7. The process according to one of the preceding claims, wherein, in step (ii), at most 20% by weight of the mixture comprising sodium formate is taken off, based on the total weight of the mixture comprising sodium formate.

8. The process according to one of the preceding claims, wherein, of the mixture comprising sodium formate obtained in step (ii), a part is taken off and ejected, wherein the amount of sodium formate present in the remaining part of the mixture together with the amount of sodium formate present in the subquantity (A) of the mother liquor gives the total amount of sodium formate used for producing the aqueous solution.

9. The process according to one of the preceding claims, wherein the water content of the mixture comprising sodium formate from step (ii), before its use in the production of the aqueous solution, is decreased to at most 20% by weight, based on the total weight of the mixture.

10. The process according to claim 9, wherein the water content is decreased by means of an evaporation stage, or, by means of a second crystallization stage in which a second solid phase and a second mother liquor are obtained, and a second concentration stage in which the second solid phase is separated off from the second mother liquor.

11. The process according to one of claims 1 to 9, in which
a) a stream (1) of formic acid having a formic acid content of at least 74% by weight is prepared;
b) the stream (1) from step a) having two streams (5a) and (10) comprising the sodium formate is fed to a crystallization stage, wherein, optionally with temperature elevation, the aqueous solution which has a molar ratio of HCOOH:Na[HCOO] of greater than 1.5:1 and a molar ratio of HCOOH:H₂O of at least 1.1:1 is produced;
c) in the crystallization stage the aqueous solution from step b) is brought to crystallization to produce a stream (3) having the solid phase and the mother liquor;
d) the stream (3) from step c) is fed to a separation stage in which the solid phase is separated off from the mother liquor, a stream (4) comprising the sodium diformate and a stream (5) comprising the mother liquor being produced;
e) the stream (5) from step d) is divided into two substreams (5a) and (5b);
f) the stream (5a) from step e) is recirculated as subquantity (A) to step b);
g) the stream (5b) from step e) is fed to a neutralization stage as subquantity (B) together with a stream (6) comprising the sodium-containing base, resulting in the mixture comprising sodium formate; and
h) the mixture from step g) comprising sodium formate, optionally after taking off a part of same in the form of stream (7a), is fed as stream (7) to a concentration stage in which a part of the water present in the stream (7) is discharged as stream (9), the stream (10) comprising sodium formate being produced, which is recirculated to the step b).

12. The process according to claim 11, wherein in step d) the stream (5) essentially comprises formic acid in the range from 35 to 80% by weight, sodium formate in the range from 20 to 45% by weight, and water in the range from 0 to 30% by weight, in each case based on the total weight of the stream (5).

13. The process according to one of claims 11 or 12, wherein, in step g), as stream (6), an aqueous caustic soda solution having an NaOH content in the range from 10 to 60% by weight, based on the total weight of the aqueous caustic soda solution, is used.

14. The process according to one of claims 11 to 13, wherein the stream (10) from step h) essentially comprises sodium formate in the range form 50 to 100% by weight and water in the range from 0 to 50% by weight, in each case based on the total weight of the stream (10).

15. The process according to one of claims 11 to 14, in which, in addition,
k) the sodium formate-comprising stream (10) from step h), before it is recirculated to step b) is fed to a second crystallization stage and is brought herein to crystallization, producing a second solid phase and a second mother liquor;
l) the second solid phase and second mother liquor obtained from step k) is fed in the form of a stream (12) to a separation stage in which the second solid phase is separated off from the second mother liquor, producing a stream (13) comprising the second mother liquor and a stream (14) comprising sodium formate;
m) the sodium formate-comprising stream (14) from step I) is recirculated to the step b) and used herein as stream (10); and
n) the mother liquor-comprising stream (13) from step I)
n1) is recirculated to the step h) and herein is fed together with the stream (7) to the concentration stage of the step h);
n2) is recirculated to the step k) and herein is fed together with the stream (10) to the second crystallization stage;
n3) is divided into the substreams (13a) and (13b), the substream (13a) is recirculated to the step h) and herein is fed together with the stream (7) to the concentration stage of the step h) and the substream (13b) is recirculated to the step k) and herein is fed together with the stream (10) to the second crystallization stage; and/or
n4) is in part taken off and ejected.

16. The process according to claim 15, wherein the stream (13) from step I) comprising the mother liquor essentially comprises water in the range from 20 to 50% by weight and sodium formate in the range from 50 to 80% by weight, in each case based on the total weight of the stream (13).

17. The process according to one of claims 15 or 16, wherein, in step n4), at most 30% by weight of the stream (13), based on the total weight of the stream (13), is taken off and ejected.

18. The process according to one of claims 15 to 17, wherein the stream (13) from step I) comprising the mother liquor is recirculated according to step n1) to step h) and herein fed together with the stream (7) to the concentration stage.

19. The process according to one of claims 15 to 18, wherein the stream (14) from step I) comprising sodium formate essentially comprises sodium formate in the range from 75 to 100% by weight and water in the range from 0 to 25% by weight, in each case based on the total weight of the stream (14).

20. The process according to one of the preceding claims, wherein the solid sodium diformate preparation has a formic acid content in the range from 38 to 41% by weight, based on the total weight of the sodium diformate preparation.

21. The process according to one of the preceding claims, wherein the solid sodium diformate preparation has a water content of no greater than 0.5% by weight, based on the total weight of the preparation.

## Revendications

1. Procédé de fabrication d'une préparation solide de diformiate de sodium ayant une teneur en acide formique d'au moins 35 % en poids, par rapport au poids total de la préparation de diformiate de sodium, selon lequel une solution aqueuse, qui présente un rapport molaire HCOOH:HCOONa supérieur à 1,5:1 et un rapport molaire HCOOH:H₂O d'au moins 1,1:1, est fabriquée à température élevée à partir de formiate de sodium et d'au moins 74 % en poids d'acide formique, la solution aqueuse est faite cristalliser et la phase solide est séparée de la liqueur mère, dans lequel
(i) une partie (A) de la liqueur mère est utilisée lors de la fabrication de la solution aqueuse et
(ii) une partie (B) de la liqueur mère est mélangée avec une base contenant du sodium et le mélange contenant du formiate de sodium résultant est également utilisé lors de la fabrication de la solution aqueuse, éventuellement après évacuation d'une partie de celui-ci et éventuellement après concentration ;
les parties (A) et (B) de la liqueur mère se complétant à 100 % en poids.

2. Procédé selon la revendication 1, dans lequel la fabrication de la solution aqueuse à lieu à une température inférieure ou égale à 100 °C.

3. Procédé selon l'une quelconque des revendications 1 ou 2, dans lequel le rapport en poids entre la partie (A) et la partie (B) de la liqueur mère est situé dans la plage allant de 20:1 à 1:10.

4. Procédé selon l'une quelconque des revendications précédentes, dans lequel le rapport entre la partie (A) et la partie (B) de la liqueur mère est ajusté de manière à ce que le rapport molaire entre HCOOH dans la partie (B) de la liqueur mère et la totalité du diformiate de sodium contenu et du formiate de sodium éventuellement contenu dans la phase solide obtenue, avant un séchage ultérieur éventuel de la phase solide, soit d'au plus 1,2:1.

5. Procédé selon l'une quelconque des revendications précédentes, dans lequel la base contenant du sodium à l'étape (ii) est choisie parmi l'hydroxyde de sodium, le carbonate de sodium, l'hydrogénocarbonate de sodium, les alcanolates en C₁-C₆ de sodium et leurs mélanges.

6. Procédé selon l'une quelconque des revendications précédentes, dans lequel la partie (B) de la liqueur mère est neutralisée essentiellement en totalité à l'étape (ii).

7. Procédé selon l'une quelconque des revendications précédentes, dans lequel au plus 20 % en poids du mélange contenant du formiate de sodium, par rapport au poids total du mélange contenant du formiate de sodium, est soutiré à l'étape (ii).

8. Procédé selon l'une quelconque des revendications précédentes, dans lequel une partie du mélange contenant du formiate de sodium obtenu à l'étape (ii) est soutirée et évacuée, la quantité de formiate de sodium contenue dans la partie restante du mélange et la quantité de formiate de sodium contenue dans la partie (A) de la liqueur mère formant ensemble la quantité totale de formiate de sodium utilisée pour la fabrication de la solution aqueuse.

9. Procédé selon l'une quelconque des revendications précédentes, dans lequel la teneur en eau du mélange contenant du formiate de sodium de l'étape (ii) est réduite à au plus 20 % en poids, par rapport au poids total du mélange, avant son utilisation lors de la fabrication de la solution aqueuse.

10. Procédé selon la revendication 9, dans lequel la teneur en eau est réduite par une étape d'évaporation ou réduite par une seconde étape de cristallisation, lors de laquelle une seconde phase solide et une seconde liqueur mère sont obtenues, et une seconde étape de concentration, lors de laquelle la seconde phase solide est séparée de la seconde liqueur mère.

11. Procédé selon l'une quelconque des revendications 1 à 9, dans lequel
a) un courant (1) d'acide formique ayant une teneur en acide formique d'au moins 74 % en poids est préparé ;
b) le courant (1) de l'étape a) est introduit dans une étape de cristallisation avec deux courants contenant le formiate de sodium (5a) et (10), la solution aqueuse, qui présente un rapport molaire HCOOH:Na [HCOO] supérieur à 1,5:1 et un rapport molaire HCOOH:H₂O d'au moins 1,1:1, étant obtenue, éventuellement avec élévation de la température ;
c) lors de l'étape de cristallisation, la solution aqueuse de l'étape b) est faite cristalliser avec obtention d'un courant (3) comprenant la phase solide et la liqueur mère ;
d) le courant (3) de l'étape c) est introduit dans une étape de séparation, lors de laquelle la phase solide est séparée de la liqueur mère, un courant (4) contenant le diformiate de sodium et un courant (5) contenant la liqueur mère étant obtenus ;
e) le courant (5) de l'étape d) est divisé en deux courants partiels (5a) et (5b) ;
f) le courant (5a) de l'étape e) est recyclé en tant que partie (A) dans l'étape b) ;
g) le courant (5b) de l'étape e) est introduit dans une étape de neutralisation en tant que partie (B) avec un courant (6) qui contient une base contenant du sodium, le mélange contenant du formiate de sodium étant obtenu ; et
h) le mélange contenant du formiate de sodium de l'étape g) , éventuellement après soutirage d'une partie de celui-ci sous la forme du courant (7a), est introduit en tant que courant (7) dans une étape de concentration, lors de laquelle une partie de l'eau contenue dans le courant (7) est déchargée sous la forme d'un courant (9), le courant (10) contenant du formiate de sodium étant obtenu, qui est recyclé dans l'étape b).

12. Procédé selon la revendication 11, dans lequel le courant (5) à l'étape d) contient principalement de l'acide formique dans la plage allant de 35 à 80 % en poids, du formiate de sodium dans la plage allant de 20 à 45 % en poids et de l'eau dans la plage allant de 0 à 30 % en poids, à chaque fois par rapport au poids total du courant (5).

13. Procédé selon l'une quelconque des revendications 11 ou 12, dans lequel une solution aqueuse d'hydroxyde de sodium ayant une teneur en NaOH dans la plage allant de 10 à 60 % en poids, par rapport au poids total de la solution aqueuse d'hydroxyde de sodium, est utilisée en tant que courant (6) à l'étape g).

14. Procédé selon l'une quelconque des revendications 11 à 13, dans lequel le courant (10) de l'étape h) contient principalement du formiate de sodium dans la plage allant de 50 à 100 % en poids et de l'eau dans la plage allant de 0 à 50 % en poids, à chaque fois par rapport au poids total du courant (10).

15. Procédé selon l'une quelconque des revendications 11 à 14, dans lequel en plus
k) le courant (10) contenant du formiate de sodium de l'étape h) est introduit dans une seconde étape de cristallisation avant le recyclage dans l'étape b) et y est fait cristalliser avec obtention d'une seconde phase solide et d'une seconde liqueur mère ;
l) la seconde phase solide et la seconde, liqueur mère obtenues à l'étape k) sont introduites sous la forme d'un courant (12) dans une étape de séparation, lors de laquelle la seconde phase solide est séparée de la seconde liqueur mère, un courant (13) contenant la seconde liqueur mère et un courant (14) contenant du formiate de sodium étant obtenus ;
m) le courant (14) contenant du formiate de sodium de l'étape 1) est recyclé dans l'étape b) et y est utilisé en tant que courant (10) ; et
n) le courant (13) contenant la liqueur mère de l'étape 1)
n1) est recyclé dans l'étape h) et y est introduit avec le courant (7) dans l'étape de concentration de l'étape h) ;
n2) est recyclé dans l'étape k) et y est introduit avec le courant (10) dans la seconde étape de cristallisation ;
n3) est divisé en courants partiels (13a) et (13b), le courant partiel (13a) étant recyclé dans l'étape h) et y étant introduit avec le courant (7) dans l'étape de concentration de l'étape h) et le courant partiel (13b) étant recyclé dans l'étape k) et y étant introduit avec le courant (10) dans la seconde étape de cristallisation ; et/ou
n4) est partiellement soutiré et évacué.

16. Procédé selon la revendication 15, dans lequel le courant (13) contenant la liqueur mère de l'étape 1) contient principalement de l'eau dans la plage allant de 20 à 50 % en poids et du formiate de sodium dans la plage allant de 50 à 80 % en poids, à chaque fois par rapport au poids total du courant (13).

17. Procédé selon l'une quelconque des revendications 15 ou 16, dans lequel au plus 30 % en poids du courant (13), par rapport au poids total du courant (13), est soutiré et évacué à l'étape n4).

18. Procédé selon l'une quelconque des revendications 15 à 17, dans lequel le courant (13) contenant la liqueur mère de l'étape 1) est recyclé selon l'étape n1) dans l'étape h) et y est introduit avec le courant (7) dans l'étape de concentration.

19. Procédé selon l'une quelconque des revendications 15 à 18, dans lequel le courant (14) contenant du formiate de sodium de l'étape 1) contient principalement du formiate de sodium dans la plage allant de 75 à 100 % en poids et de l'eau dans la plage allant de 0 à 25 % en poids, à chaque fois par rapport au poids total du courant (14).

20. Procédé selon l'une quelconque des revendications précédentes, dans lequel la préparation solide de diformiate de sodium présente une teneur en acide formique dans la plage allant de 38 à 41 % en poids, par rapport au poids total de la préparation de diformiate de sodium.

21. Procédé selon l'une quelconque des revendications précédentes, dans lequel la préparation solide de diformiate de sodium présente une teneur en eau inférieure ou égale à 0,5 % en poids, par rapport au poids total de la préparation.
